# EUROPEAN PATENT APPLICATION

(11) **EP 3 778 591 A1**
(43) Date of publication of application: **17.02.2021**
(21) Application number: 19784758.5
(22) Date of filing: 12.04.2019
(51) Int. Cl.: C07D 403/04, C07D 207/33, C07C 15/42

(54) **METHOD FOR PREPARING PYRROLOAMINOPYRIDAZINONE COMPOUND AND INTERMEDIATES THEREOF**

(30) Priority: 13.04.2018 CN 201810328604
(71) Applicant: Jiangsu Hengrui Medicine Co., Ltd., Lianyungang, Jiangsu 222047 (CN); Shanghai Shengdi Pharmaceutical Co., Ltd, Shanghai 201210 (CN)
(72) Inventor: ZHU, Lingjian, Shanghai 201210 (CN); GUAN, Zhongjun, Shanghai 201210 (CN); JIANG, Wei, Shanghai 201210 (CN); HUANG, Jian, Shanghai 201210 (CN)
(74) Representative: Potter Clarkson
(86) International application number: PCT/CN2019/082367
(87) International publication number: WO 2019/196915

(57) **Abstract**

The present invention relates to a method for preparing a pyrroloaminopyridazinone compound and intermediates thereof. Specifically relating to a method for preparing the compound of formula (I), the target product being prepared by means of changing the starting materials and intermediates; the present method has the advantages of reactants such as the starting materials being easy to purchase, the reaction conditions being simple and controllable, the post-reaction treatment method being simple, the yield being high, and being beneficial for industrial production.

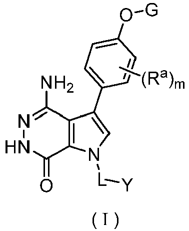

## Description

### FIELD OF THE INVENTION

The present invention relates to a method for preparing pyrroloaminopyridazinone compound and intermediates thereof.

### BACKGROUND OF THE INVENTION

Immune cells are generally classified into T cells and B cells, wherein the main function of B cells is to secrete various antibodies to protect the body against various kinds of foreign invasion. Bruton tyrosine protein kinase (BTK) is a member of the tyrosine protein kinase subfamily, and belongs to the Tec family kinase. It is mainly expressed in B cells, and distributed in the lymphatic system, hematopoietic and hematological systems. B-cell receptor (BCR) plays a crucial role in regulating the proliferation and survival of various lymphomas including subtypes of chronic lymphocytic leukemia (CLL) and non-Hodgkin lymphoma (NHL), mantle cell lymphoma (MCL) and diffuse large B-cell lymphoma (DLBCL). In addition, the effects of B cells on the pathogenesis of rheumatoid arthritis, systemic lupus erythematosus, multiple sclerosis and other immune diseases have been proven in clinical practice. Bruton tyrosine protein kinase (BTK) is a key protein kinase in the BCR signaling pathway. It is capable of regulating the maturation and differentiation of normal B cells, and is also closely related to various diseases of B cell lymphoid tissue disorders. Therefore, the small molecule inhibitor targeting BTK can be beneficial to the treatment of B-cell malignancies and autoimmune diseases.

WO2016007185A1 relates to a compound of formula (Ia), *i.e.,* (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-*d*]pyridazin-7-one. This compound is a novel BTK kinase inhibitor, and has improved kinase selectivity, clinical efficacy or indications, and safety. The structure of the compound is as following:

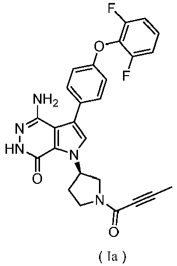

Example 1, intermediate 2 and Example 93 of WO2016007185A1 disclosed a method for preparing the compound comprising a total of ten steps, and the reaction scheme is illustrated as following:

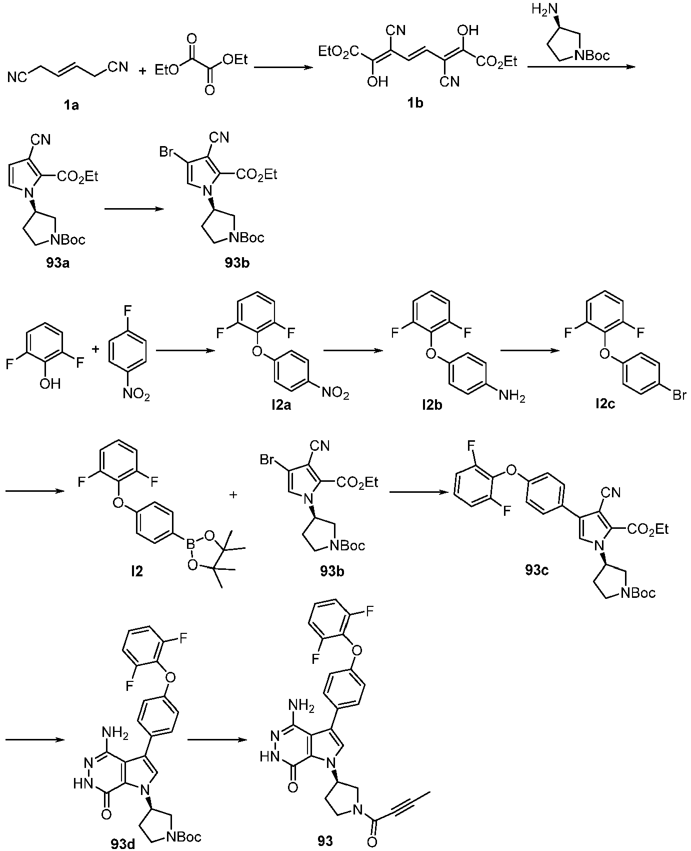

In this method, the yield of compound 93c is merely 22.8%, and the yield of product 93 is merely 51%. The whole method has the problem that several steps in the method have a low yield and difficulty in purification, leading to a low total yield and poor feasibility of industrial production. Moreover, palladium catalyst is used in the method, resulting in a high cost. Therefore, it is necessary to improve the existing preparation method.

### SUMMARY OF THE INVENTION

The technical problem to be solved by the present invention is to provide a method for preparing a compound of formula (I) that is different from the prior art. The preparation method is optimized by the ways such as changing the starting materials and intermediates to the reactants that are simple and easy to purchase, changing the reaction conditions to the simple and controllable ones, and simplifing the work-up process, which can improvide the yield and is conducive to industrial production.

The technical solutions of the present invention are as follows.

The present invention provides a compound of formula (E), a salt thereof, or a stereoisomer thereof, 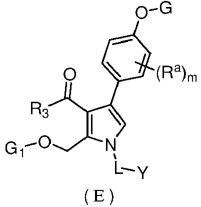 wherein,
R^{a} is selected from the group consisting of hydrogen, halogen, hydroxy, nitro, cyano, carboxy, amino, alkyl, haloalkyl, haloalkoxy and alkoxy;
R₃ is selected from the group consisting of hydrogen, halogen, alkyl, -OR₁, -NHR₂, -NR₂R₂ and alkylsulfonamido;
R₁ is selected from the group consisting of hydrogen, alkyl, alkylcarbonyl, cycloalkyl, cycloalkylcarbonyl, heterocyclyl, heterocyclylcarbonyl, aryl, arylcarbonyl, heteroaryl and heteroarylcarbonyl;
R₂ is selected from the group consisting of hydrogen, alkyl, alkylamino, alkylcarbonyl, alkoxycarbonyl, alkylaminocarbonyl, cycloalkyl, cycloalkylcarbonyl, heterocyclyl, heterocyclylcarbonyl, aryl, arylcarbonyl, heteroaryl and heteroarylcarbonyl;
G is selected from the group consisting of aryl, heteroaryl, cycloalkyl and heterocyclyl, which are optionally substituted by a substituent, wherein the substituent is selected from the group consisting of hydrogen, halogen, hydroxy, nitro, cyano, carboxy, amino, alkyl, alkoxy, alkylamino, hydroxyalkyl, dialkylamino, alkylcarbonyl, formylalkyl, alkoxycarbonyl, formylalkoxy, alkylcarbonylamino, alkylaminocarbonyl, alkylsulfonyl, alkenyl, alkenylcarbonyl, alkynyl and alkynylcarbonyl;
G₁ is selected from the group consisting of hydrogen and a hydroxy protecting group, the hydroxy protecting group is preferably a methyl, 9-fluorenylmethyl, triisopropylsilylmethyl, cyclopropylmethyl, diphenylmethyl, triphenylmethyl, ethyl, 2,2,2-trichloroethyl, 2-(trimethylsilyl)ethyl, 2-(*p*-toluenesulfonyl)ethyl, 2-cyanoethyl, allyl, *tert*-butyl, methoxymethyl, benzyloxymethyl, triisopropylsiloxymethyl, 2,6-dimethylphenyl, 2,6-diisopropylphenyl, 2,6-di-*tert*-butyl-4-methoxyphenyl, benzyl, *p*-methylbenzyl, 2,4-dimethoxybenzyl, 2,6-dimethoxybenzyl, *p*-nitrobenzyl, *o*-nitrobenzyl, trimethylsilyl, triethylsilyl, triisopropylsilyl, phenyldimethylsilyl or trimethyl stannyl;
L is an alkylene or absent;
Y is selected from the group consisting of cycloalkyl, heterocyclyl, aryl and heteroaryl, which are optionally substituted by a substituent, wherein the substituent is selected from the group consisting of halogen, cyano, alkylcarbonyl, alkoxycarbonyl, alkylcarbonylamino, alkylsulfonyl, alkylsulfonamido, alkyl, cycloalkyl, alkenyl, alkenylcarbonyl, alkynyl and alkynylcarbonyl, Y is preferably a 3 to 8 membered heterocyclyl, and more preferably pyrrolidinyl or piperidinyl;
m is 0, 1, 2 or 3.

Preferably, the compound is a compound of formula (E1) or (E2) 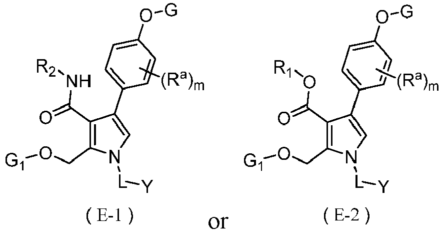 wherein R^{a}, R₁, R₂, G, G₁, L, Y and m are as defined in formula (E).

More preferably, the compound is selected from the group consisting of

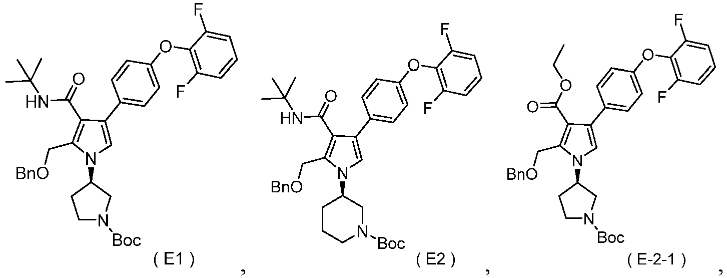

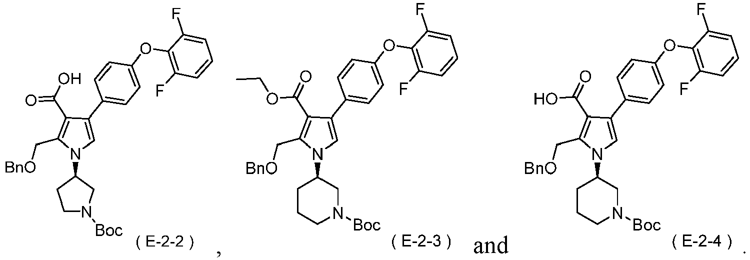

The present invention also provides a method for preparing the compound of formula (E) or a stereoisomer thereof, characterized in that the method comprises a step of reacting a compound of formula (SM1) or a stereoisomer thereof, a compound of formula (SM2) or a stereoisomer thereof and a compound of formula (SM3) or a stereoisomer thereof to obtain the compound of formula (E) or a stereoisomer thereof, 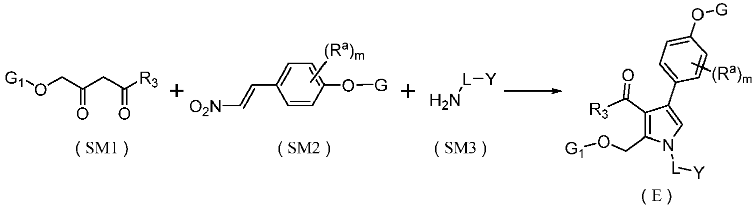 wherein R^{a}, R₃, G, G₁, L, Y and m are as defined in formula (E).

In the above embodiments, when R₃ is not -OR₁, the method may further comprise a step of:

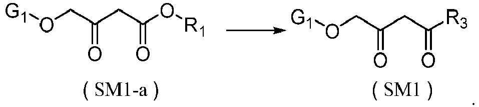

In the above embodiments, the method further comprises a step of

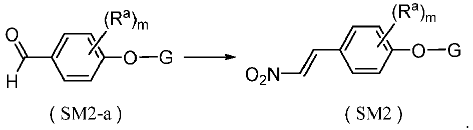

The present invention also provides a method for preparing the compound of formula (E1) or a stereoisomer thereof, characterized in that the method comprises a step of

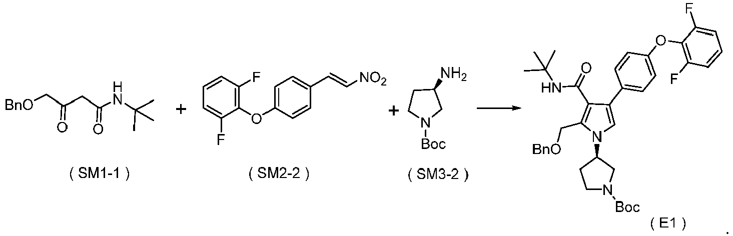

In the above embodiments, the method further comprises a step of

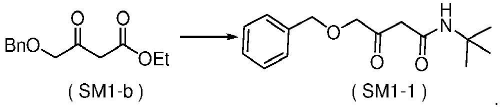

In the above embodiments, the method further comprises a step of

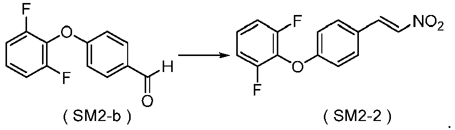

The present invention also provides a method for preparing the compound of formula (E-2-1) or a stereoisomer thereof, characterized in that the method comprises a step of

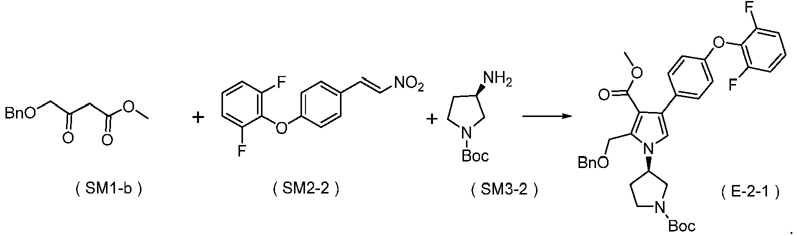

In the above embodiments, the method further comprises a step of

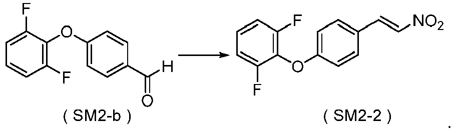

In the above embodiments, the method further comprises a step of

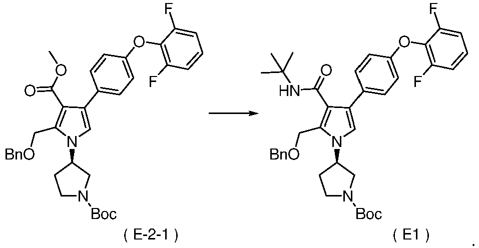

In the above embodiments, the method further comprises a step of

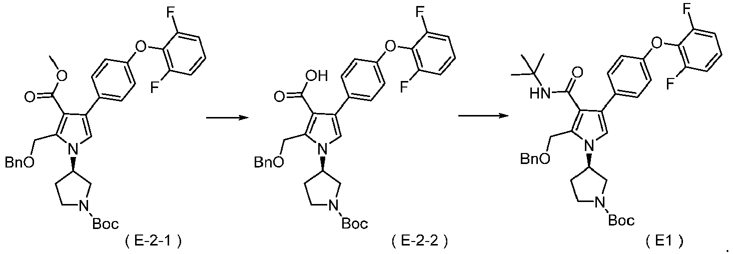

The present invention also provides a compound of formula (SM1), a salt thereof, or a stereoisomer thereof, 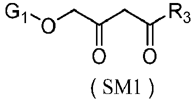 wherein R₃ and G₁ are as defined in formula (E).

Preferably, the compound is

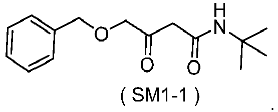

The present invention also provides a compound of formula (SM2), a salt thereof, or a stereoisomer thereof, 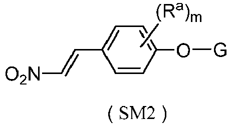 wherein R^{a}, G and m are as defined in formula (E).

Preferably, the compound is

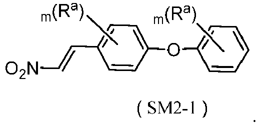

More preferably, the compound is

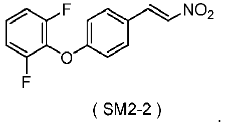

The present invention also provides a method for preparing the compound of formula (SM1) or a stereoisomer thereof, characterized in that the method comprises a step of reacting a compound of formula (SM1-a) to obtain the compound of formula (SM1) or a stereoisomer thereof, 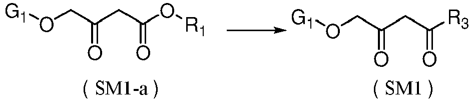 wherein R₁, R₃ and G₁ are as defined in formula (E), and R₃ is not -OR₁.

In a preferred embodiment of the present invention, the method comprises a step of

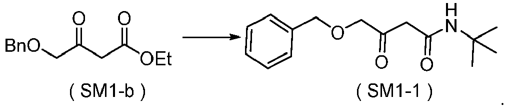

The present invention also provides a method for preparing the compound of formula (SM2) or a stereoisomer thereof, characterized in that the method comprises a step of reacting a compound of formula (SM2-a) or a stereoisomer thereof to obtain the compound of formula (SM2) or a stereoisomer thereof, 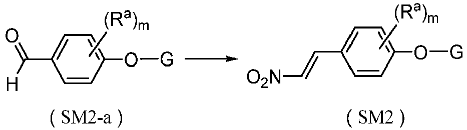 wherein R^{a}, G and m are as defined in formula (E).

In a preferred embodiment of the present invention, the method comprises a step of

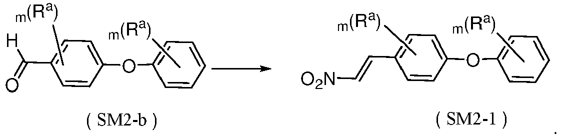

In a preferred embodiment of the present invention, the method comprises a step of

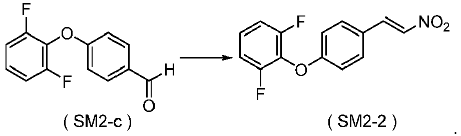

The present invention also provides a compound of formula (B), a salt thereof, or a stereoisomer thereof, 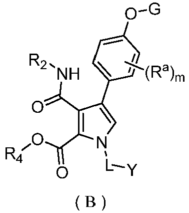 wherein R^{a}, R₂, G, L, Y and m are as defined in formula (E);

R₄ is selected from the group consisting of hydrogen, alkyl, alkylcarbonyl, alkoxycarbonyl, alkylaminocarbonyl, alkylsulfonyl, cycloalkyl, heterocyclyl, aryl and heteroaryl.

Preferably, the compound is selected from the group consisting of

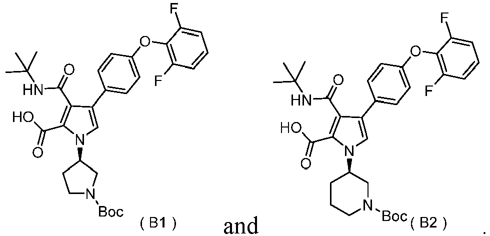

The present invention also provides a compound of formula (C), a salt thereof, or a stereoisomer thereof, 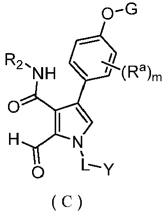 wherein R^{a}, R₂, G, L, Y and m are as defined in formula (E).

Preferably, the compound is selected from the group consisting of

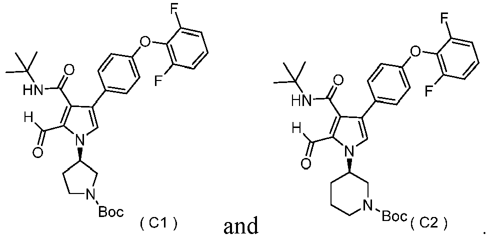

The present invention also provides a method for preparing the compound of formula (B) or a stereoisomer thereof, characterized in that the method comprises a step of 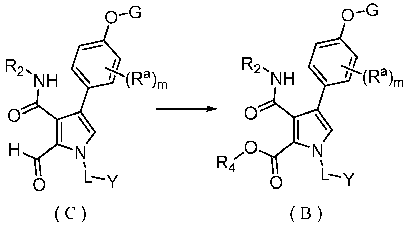 wherein R^{a}, R₂, G, L, Y and m are as defined in formula (E); and R₄ is as defined in formula (B).

In the above embodiments, the method further comprises a step of

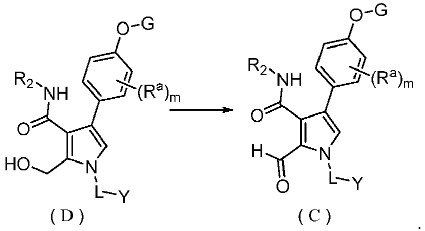

In the above embodiments, the method may further comprise the steps of 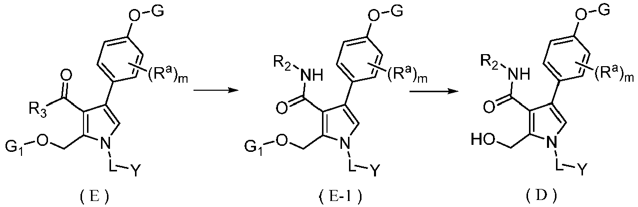 wherein G₁ and R₃ are as defined in formula (E), and R₃ is not -NHR₂.

In some embodiments, the method for preparing the compound of formula (B) or a stereoisomer thereof may further comprise a step of

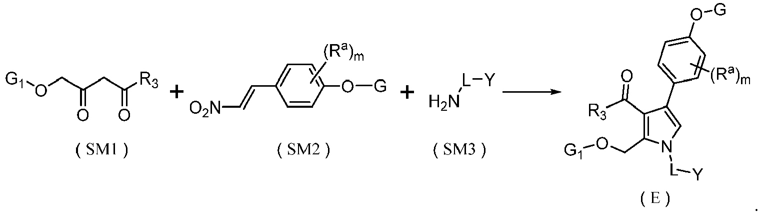

In the above embodiments, the method further comprises a step of

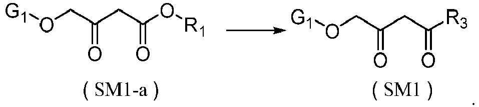

In the above embodiments, the method further comprises a step of

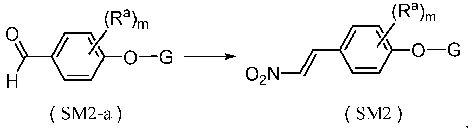

The present invention also provides a method for preparing the compound of formula (B1) or a stereoisomer thereof, characterized in that the method comprises a step of

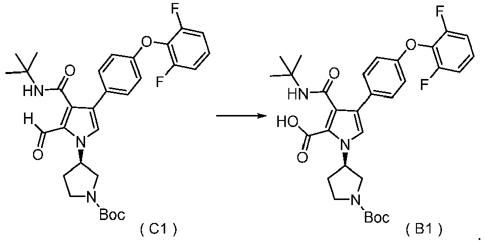

In the above embodiments, the method further comprises a step of

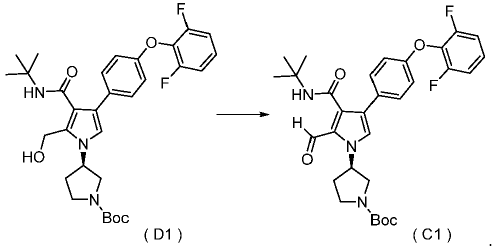

In the above embodiments, the method further comprises a step of

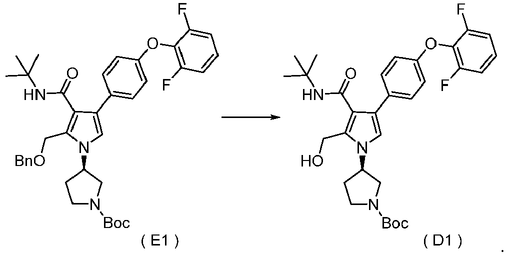

In the above embodiments, the method further comprises a step of

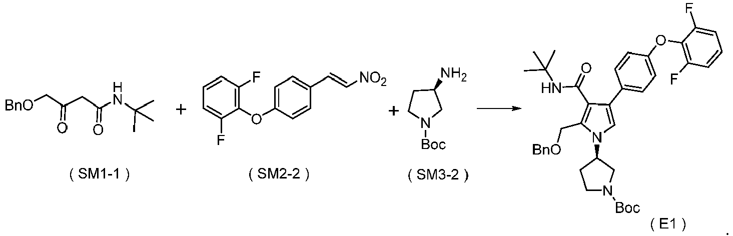

In the above embodiments, the method further comprises a step of

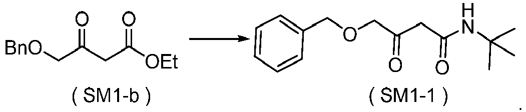

In the above embodiments, the method further comprises a step of

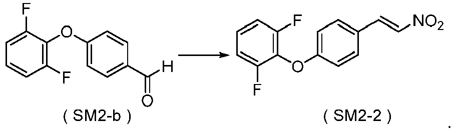

The present invention also provides a method for preparing the compound of formula (A) or a stereoisomer thereof, characterized in that the method comprises a step of 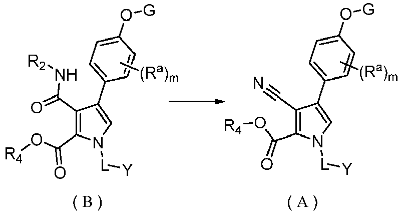 wherein R^{a}, R₂, G, L, Y and m are as defined in formula (E); and R₄ is as defined in formula (B).

In the above embodiments, the method further comprises a step of

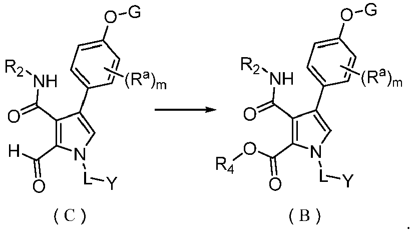

In the above embodiments, the method further comprises a step of

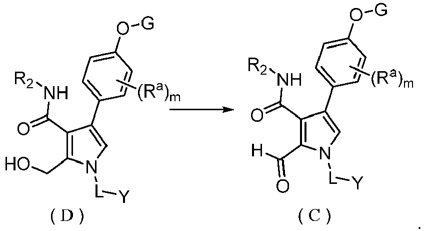

In the above embodiments, the method further comprises the steps of 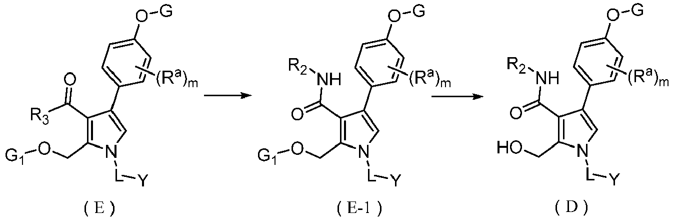 wherein G₁ and R₃ are as defined in formula (E).

In the above embodiments, the method further comprises a step of

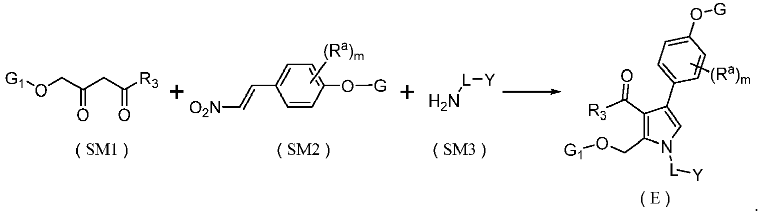

In the above embodiments, the method further comprises a step of

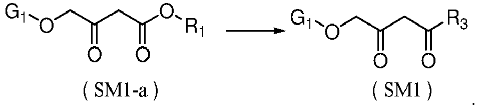

In the above embodiments, the method further comprises a step of

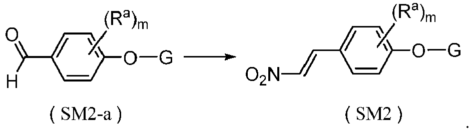

The present invention also provides a method for preparing the compound of formula (A1) or a stereoisomer thereof, characterized in that the method comprises the steps of

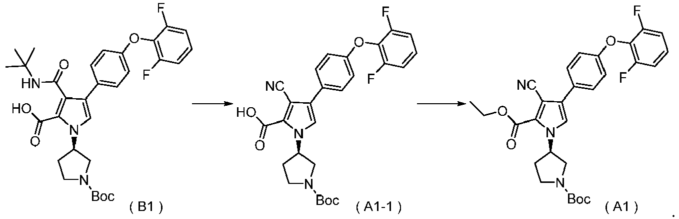

In the above embodiments, the method further comprises a step of

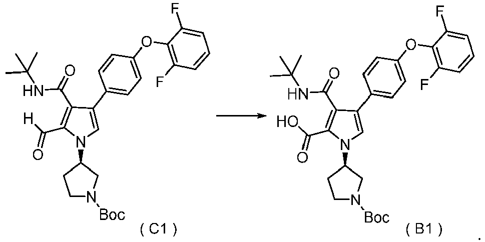

In the above embodiments, the method further comprises a step of

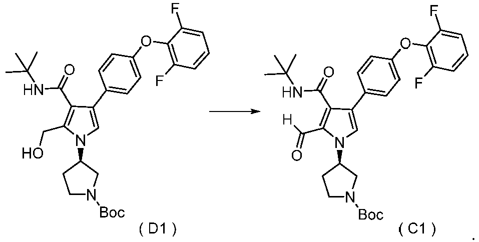

In the above embodiments, the method further comprises a step of

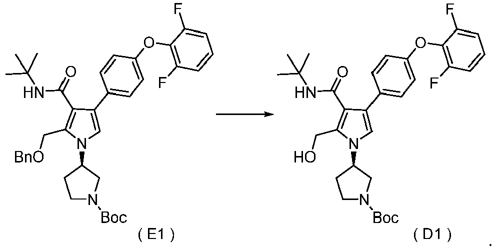

In the above embodiments, the method further comprises a step of

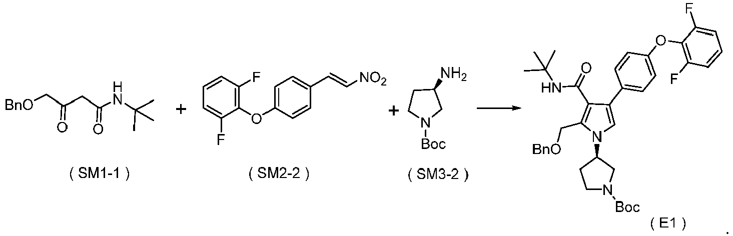

In the above embodiments, the method further comprises a step of

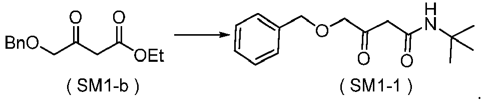

In the above embodiments, the method further comprises a step of

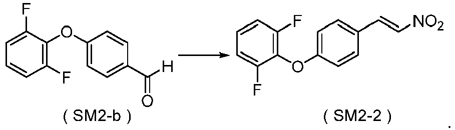

The present invention also provides a method for preparing the compound of formula (I) or a stereoisomer thereof, characterized in that the method comprises the steps of reacting a compound of formula (B) or a stereoisomer thereof to obtain a compound of formula (A) or a stereoisomer thereof, and reacting the compound of formula (A) or a stereoisomer thereof to obtain the compound of formula (I) or a stereoisomer thereof, 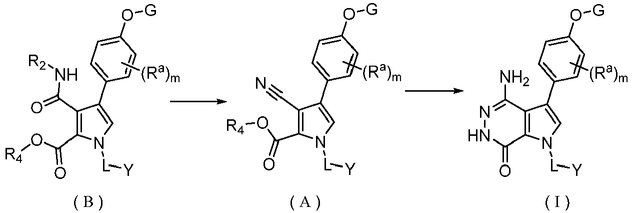 wherein R^{a}, R₂, G, L, Y and m are as defined in formula (E); and R₄ is as defined in formula (B).

In the above embodiments, the method further comprises a step of

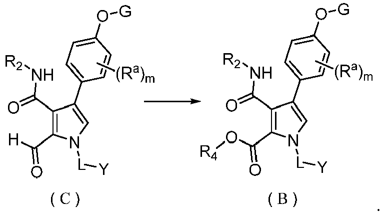

In the above embodiments, the method further comprises a step of

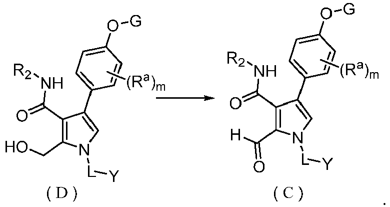

In the above embodiments, the method further comprises steps of 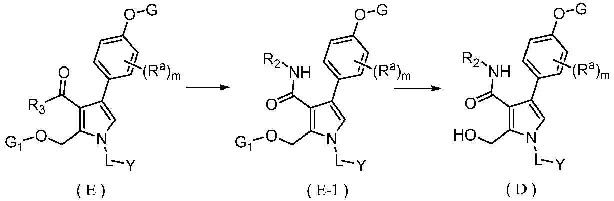 wherein G₁ and R₃ are as defined in formula (E).

In the above embodiments, the method further comprises a step of

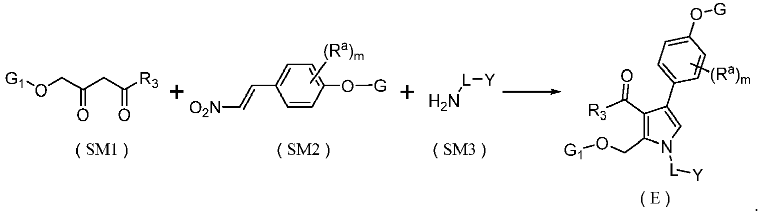

In the above embodiments, the method further comprises a step of

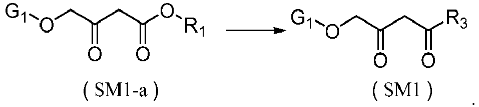

In the above embodiments, the method further comprises a step of

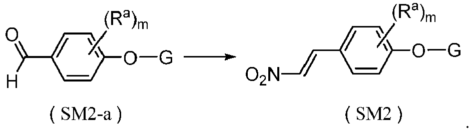

The present invention also relates to a method for preparing the compound of formula (Ia) or a stereoisomer thereof, characterized in that the method comprises steps of reacting a compound of formula (B1) to obtain a compound of formula (A1-1), reacting the compound of formula (A1-1) to obtain a compound of formula (A1), reacting the compound of formula (A1) to obtain a compound of formula (III), reacting the compound of formula (III) to obtain a compound of formula (II), and reacting the compound of formula (II) to obtain the compound of formula (Ia),

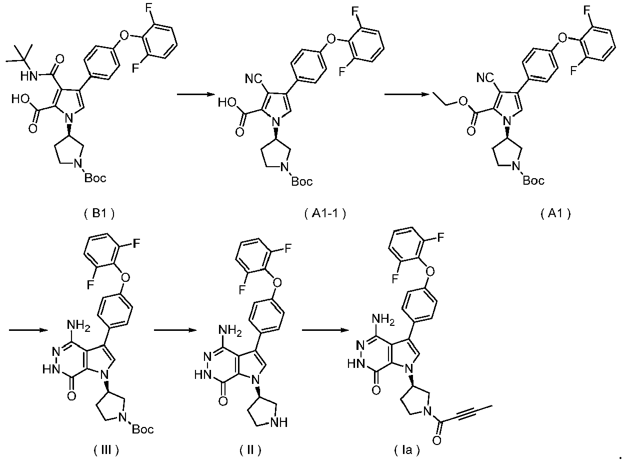

In the above embodiments, the method further comprises a step of

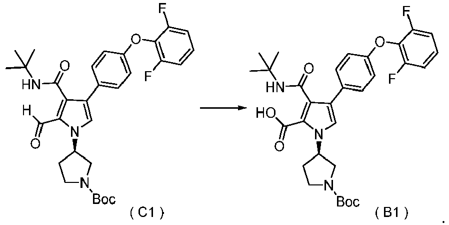

In the above embodiments, the method further comprises a step of

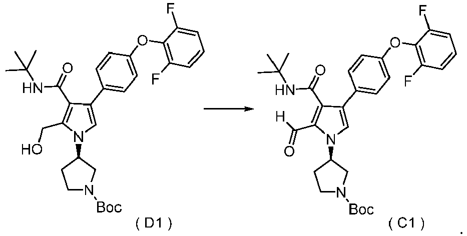

In the above embodiments, the method further comprises a step of

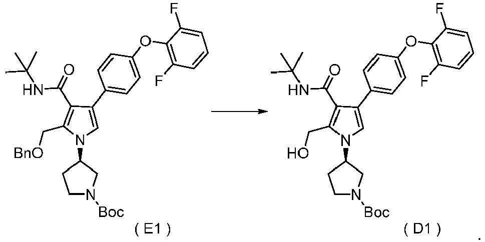

In the above embodiments, the method further comprises a step of

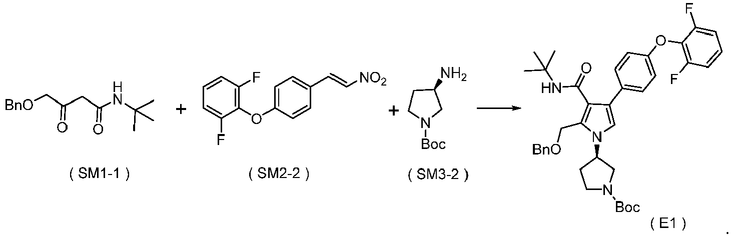

In the above embodiments, the method further comprises a step of

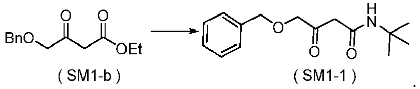

In the above embodiments, the method further comprises a step of

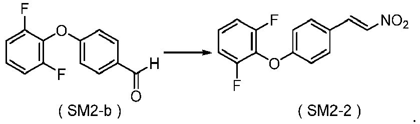

The present invention also relates to a method for preparing the compound of formula (Ia) or a stereoisomer thereof, characterized in that the method comprises steps of

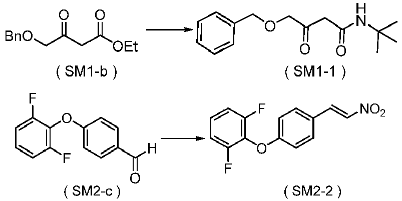

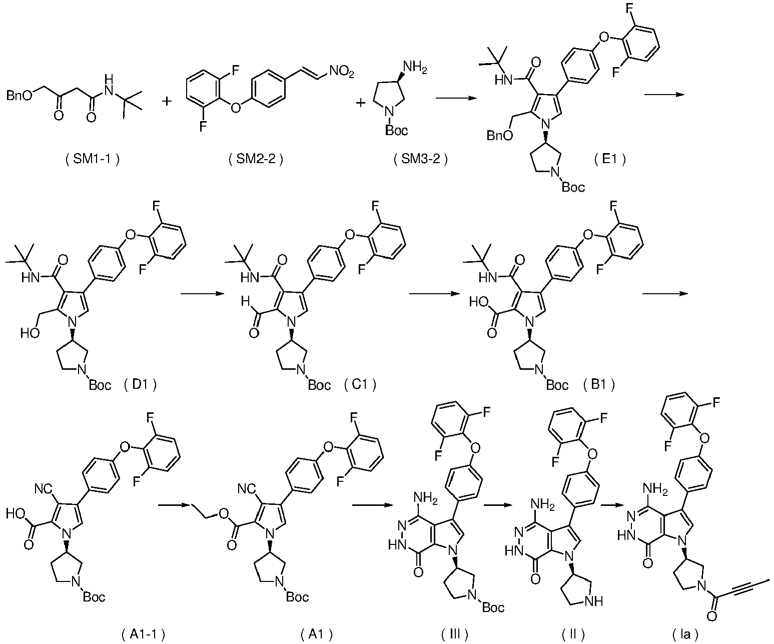

### Step 1. Preparation of the compound of formula (SM1-1)

Compound **SM1-b,** a primary amine and a base are added to a solvent. The resulting solution is heated under reflux, cooled, concentrated and dried to give the compound of formula (SM1-1). The base is selected from the group consisting of triethylamine and 4-dimethylaminopyridine.

The solvent is selected from the group consisting of tert-butanol, toluene and xylene.

### Step 2. Preparation of the compound of formula (SM2-2)

Acetic acid and a base are added to a reaction flask, and stirred to dissolve. A solution of compound **SM2-c** in nitromethane is added, and the reaction solution is heated. The reaction solution is added with water to precipitate a solid which is then filtrated out to give the compound of formula (SM2-2). The base is selected from the group consisting of ammonium acetate and piperidine.

### Step 3. Preparation of the compound of formula (E1)

Compound **SM1-1,** compound **SM3-2,** compound **SM2-2** and nitromethane are added to a solvent, and stirred at room temperature. A catalyst is added under stirring, and the reaction solution is heated to reflux and stirred. The reaction solution is cooled, concentrated and dried to give the compound of formula (E1).

The catalyst is preferably a transition metal salt catalyst, iodine or triphenylphosphine. The transition metal salt catalyst is selected from the group consisting of ferric trichloride, ferrous chloride, cuprous iodide and palladium.

The solvent is selected from the group consisting of toluene and xylene.

### Step 4. Preparation of the compound of formula (D1)

Compound **E1** is added to an alcohol solvent and stirred, followed by the one-time addition of a palladium catalyst under hydrogen atmosphere. The reaction solution is heated and stirred. After completion of the reaction, the reaction solution is cooled, filtrated and concentrated to give the compound of formula (D1).

The alcohol solvent is selected from the group consisting of methanol, ethanol, isopropanol and n-pentanol.

### Step 5. Preparation of the compound of formula (C1)

Compound **D1** is added to a solvent and stirred to dissolve, followed by the one-time addition of S-IBX. The reaction solution is heated, and cooled after completion of the reaction. Methyl *tert*-butyl ether is added and stirred. The reaction solution is filtrated, and the filtrate is extracted with methyl *tert*-butyl ether. The organic phases are combined, dried, filtrated and concentrated to obtain the compound of formula (C1).

The solvent is selected from the group consisting of dichloromethane and dimethyl sulfoxide.

### Step 6. Preparation of the compound of formula (B1)

Compound **C1,** an alcohol solvent and isopentene are added to a reaction flask, and stirred to dissolve. Sodium chlorite and sodium dihydrogen phosphate are dissolved in water, which is then added dropwise to the above reaction solution. After completion of the addition, the reaction solution is stirred at room temperature. After completion of the reaction, the reaction solution is concentrated, followed by the addition of water and extraction with methyl *tert*-butyl ether. The organic phases are combined, dried, filtrated and concentrated to give the compound of formula (B1).

The alcohol solvent is selected from the group consisting of methanol, ethanol, isopropanol and *n*-pentanol.

### Step 7. Preparation of the compound of formula (A1-1)

Compound **B1** and a halogenated hydrocarbon solvent are added to a reaction flask. Trifluoroacetic anhydride is dissolved in the halogenated hydrocarbon solvent, which is then added dropwise to the above reaction system. After completion of the addition, the reaction solution is reacted at room temperature. After completion of the reaction, water is added to the reaction solution and stirred well, and the aqueous phase is extracted with a solvent. The organic phases are combined, dried, filtrated and concentrated to give the compound of formula (A1-1).

The solvent is selected from the group consisting of dichloromethane.

### Step 8. Preparation of the compound of formula (A1)

The *tert*-butylamine salt of compound **A1-1,** potassium carbonate and an amide solvent are added to a reaction flask. Halogenated ethane or an active ester of ethanol (such as ethyl methanesulfonate and the like) is added. After completion of the addition, the reaction solution is reacted at 30°C until the raw materials are completely consumed. The reaction solution is added with water, and extracted with methyl *tert*-butyl ether. The organic phases are combined, dried, filtrated and concentrated to give the compound of formula (A1).

The amide solvent is selected from the group consisting of *N,N*-dimethylformamide.

### Step 9. Preparation of the compound of formula (III)

The compound of formula (A1) is dissolved in an organic solvent under heating, and added with 85% hydrazine hydrate The reaction solution is heated under reflux. The reaction solution is cooled and concentrated. The residues are added with water and then sujected to extraction. The combined organic phases are dried, filtrated, washed and concentrated to give the compound of formula (III). The organic solvent is selected from the group consisting of alcohol solvents, ether solvents, ketone solvents and nitrile solvents.

The alcohol solvent is selected from the group consisting of methanol, ethanol, isopropanol and *n*-pentanol.

The ether solvent is selected from the group consisting of tetrahydrofuran and 1,4-dioxane.

The ketone solvent is selected from the group consisting of *N*-methylpyrrolidone and acetone.

The nitrile solvent is selected from the group consisting of acetonitrile and propionitrile.

Acetone, tetrahydrofuran, acetonitrile, *N*-methylpyrrolidone, methanol, ethanol or isopropanol is preferred, and ethanol is more preferred.

### Step 10. Preparation of the compound of formula (II)

An organic solvent is added to a reactor, and the Boc protecting group is removed under an acidic condition. The compound of formula (III) is added under stirring, and the reaction solution is stirred at room temperature, and then concentrated and dried to give the compound of formula (II). The organic solvent is selected from the group consisting of halogenated hydrocarbon solvents, ester solvents, ether solvents and alcohol solvents. The acid is preferably hydrochloric acid, acetic acid or trifluoroacetic acid, and more preferably hydrogen chloride gas.

The halogenated hydrocarbon solvent is selected from the group consisting of dichloromethane, chloroform and carbon tetrachloride.

The ester solvent is selected from the group consisting of ethyl acetate, dimethyl phthalate and butyl acetate.

The ether solvent is selected from the group consisting of tetrahydrofuran, diethyl ether and dioxane.

The alcohol solvent is selected from the group consisting of methanol and ethanol.

Dichloromethane, ethyl acetate, tetrahydrofuran or ethanol is preferred, and ethanol is more preferred.

### Step 11. Preparation of the compound of formula (Ia)

The compound of formula (II) and 2-butynoic acid or 2-butynoyl chloride are subjected to a condensation reaction in the presence of a condensing agent. The reaction solution is added with purified water is added to the reaction solution, followed by stirring and extraction. The organic phase is washed with purified water, dried, filtrated and washed, and the filtrate is concentrated to give the compound of formula (Ia). The condensing agent is selected from the group consisting of 1-(3-dimethylaminopropyl)-3-ethylcarbodiimide hydrochloride/1-hydroxybenzotriazole, 2-(7-oxobenzotriazole)-*N,N,N',N*'-tetramethylurea hexafluorophosphate, dicyclohexylcarbodiimide/4-*N,N*-dimethylpyridine, 1-(3-dimethylaminopropyl)-3-ethylcarbodiimide hydrochloride and oxalyl chloride, and preferably 1-(3-dimethylaminopropyl)-3-ethylcarbodiimide hydrochloride.

The present invention also relates to a method for preparing the compound of formula (A1) or a stereoisomer thereof, characterized in that the method comprises the steps of

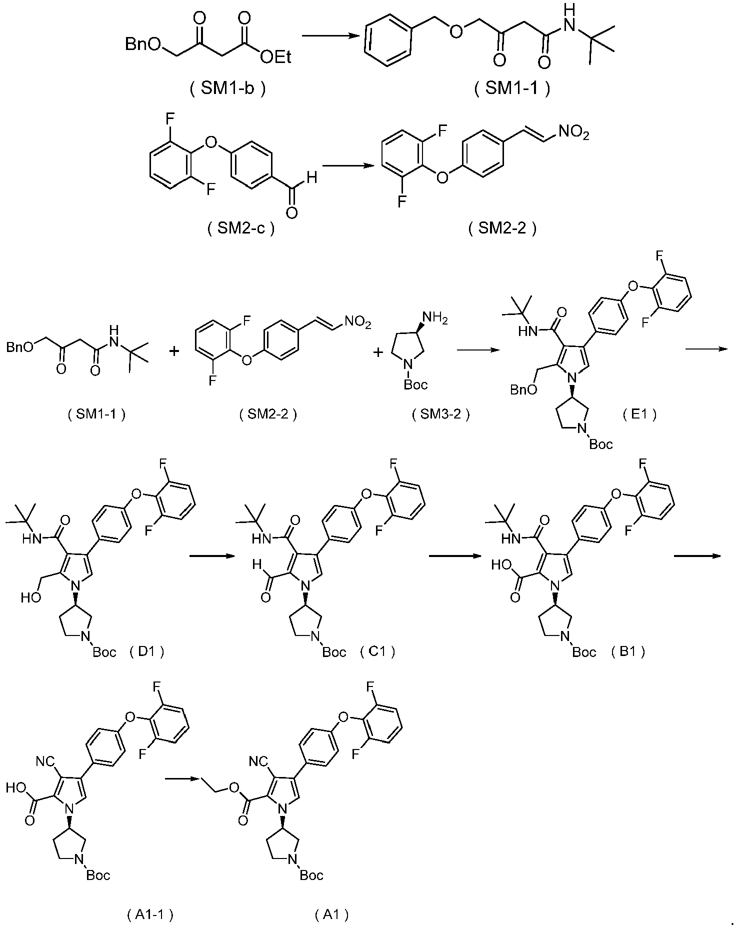

The present invention also provides a method for preparing the compound of formula (IA) or a stereoisomer thereof, characterized in that the method comprises the steps of

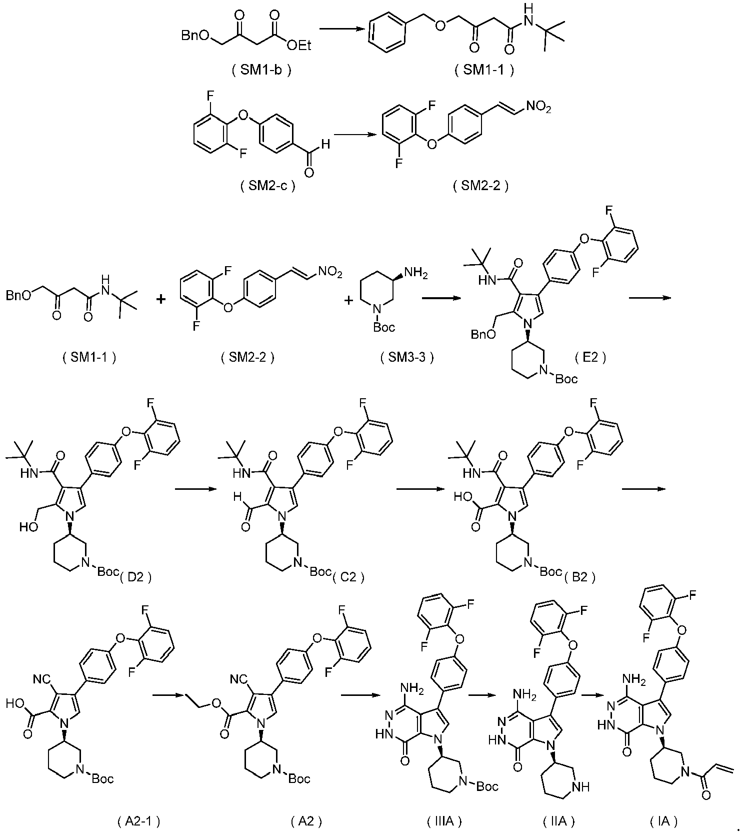

The present invention also provides a method for preparing a compound of formula (A2) or a stereoisomer thereof, characterized in that the method comprises the steps of

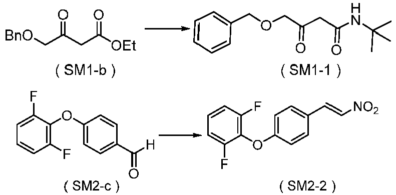

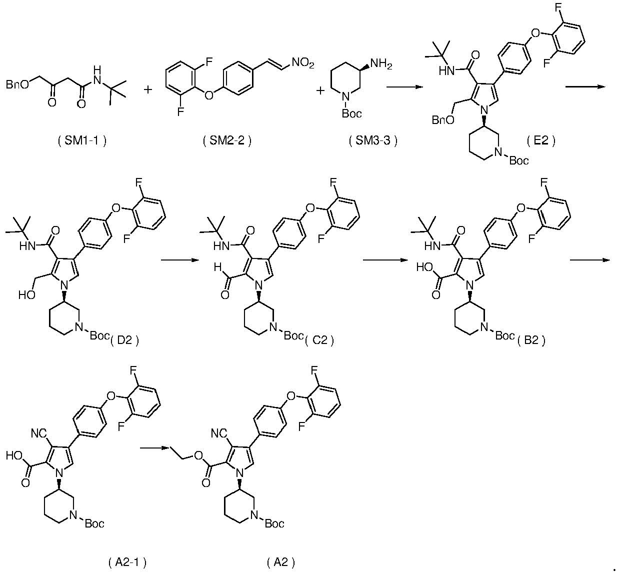

The present invention also relates to a method for preparing a compound of formula (E2) or a stereoisomer thereof, characterized in that the method comprises the steps of

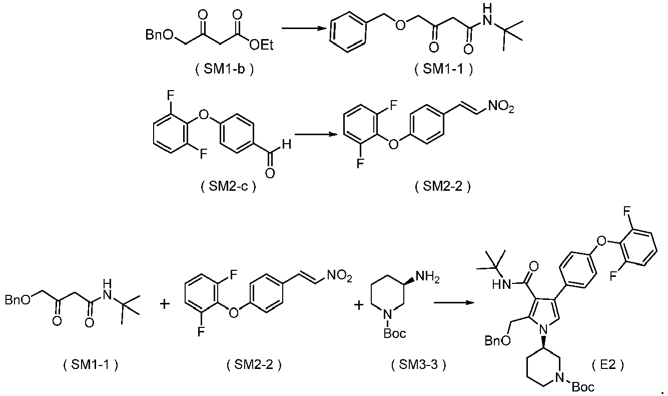

The present invention also relates to a method for preparing a compound of formula (E2) or a stereoisomer thereof, characterized in that the method comprises the steps of

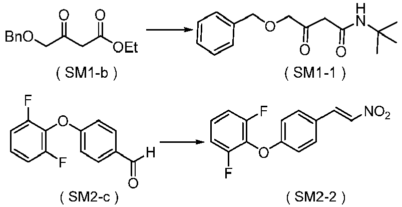

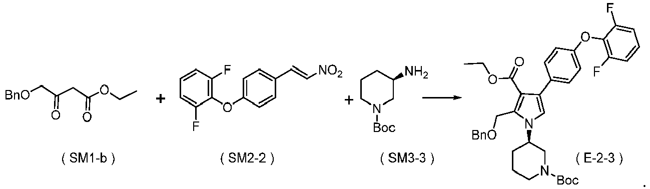

In the above embodiments, the method further comprises a step of

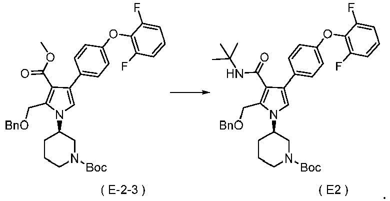

In the above embodiments, the method further comprises the steps of

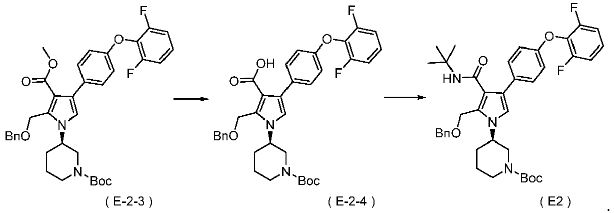

The present invention further provides a method for preparing a compound of formula (Ic) or a stereoisomer thereof, characterized in that the method comprises the steps of 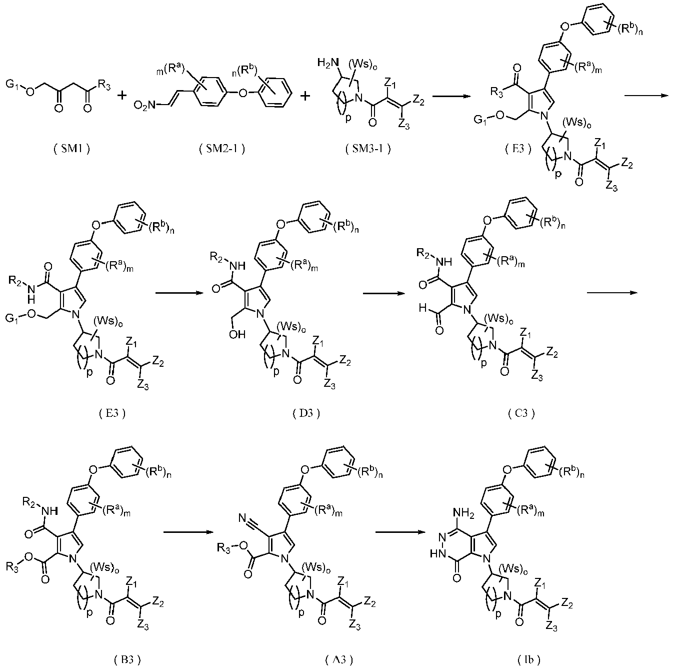 wherein,
R^{a} and R^{b} are each independently selected from the group consisting of hydrogen, halogen, hydroxy, nitro, cyano, carboxy, amino, alkyl, haloalkyl, haloalkoxy and alkoxy;
R₃ is selected from the group consisting of hydrogen, halogen, alkyl, -OR₁, -NHR₂, -NR₂R₂ and alkylsulfonamido;
R₁ is selected from the group consisting of hydrogen, alkyl, alkylcarbonyl, cycloalkyl, cycloalkylcarbonyl, heterocyclyl, heterocyclylcarbonyl, aryl, arylcarbonyl, heteroaryl and heteroarylcarbonyl;
R₂ is selected from the group consisting of hydrogen, alkyl, alkylamino, alkylcarbonyl, alkoxycarbonyl, alkylaminocarbonyl, cycloalkyl, cycloalkylcarbonyl, heterocyclyl, heterocyclylcarbonyl, aryl, arylcarbonyl, heteroaryl and heteroarylcarbonyl;
G is selected from the group consisting of aryl, heteroaryl, cycloalkyl and heterocyclyl, which are optionally substituted by a substituent, wherein the substituent is selected from the group consisting of hydrogen, halogen, hydroxy, nitro, cyano, carboxy, amino, alkyl, alkoxy, alkylamino, hydroxyalkyl, dialkylamino, alkylcarbonyl, formylalkyl, alkoxycarbonyl, formylalkoxy, alkylcarbonylamino, alkylaminocarbonyl, alkylsulfonyl, alkenyl, alkenylcarbonyl, alkynyl and alkynylcarbonyl;
G₁ is selected from the group consisting of hydrogen and a hydroxy protecting group;
Ws is selected from the group consisting of hydrogen, halogen, cyano, hydroxy, alkyl and alkoxy;
X is selected from the group consisting of fluorine, chlorine, bromine and iodine;
Z₁, Z₂ and Z₃ are each independently selected from the group consisting of hydrogen, halogen, cyano, hydroxy, amino, carboxy, alkyl, alkoxy, cycloalkyl, heterocyclyl, alkylcarbonyl, formylalkyl, alkoxycarbonyl, formylalkoxy, alkylaminocarbonyl, formylalkylamino and alkylsulfonyl, and Z₁ and Z₂ can be attached to form a bond or can form a 5 to 12 membered cycloalkyl or a 5 to 12 membered heterocyclyl together with the atom attached to them;
m = 0, 1,2 or 3;
n = 0, 1, 2 or 3;
o = 0, 1, 2 or 3; and
p = 1, 2 or 3.

Preferably, the above embodiment further comprises a step of

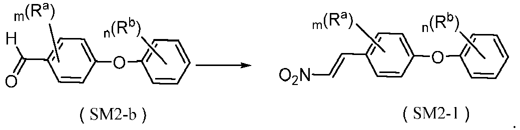

The present invention further provides a method for preparing a compound of formula (A3) or a stereoisomer thereof, characterized in that the method comprises the steps of 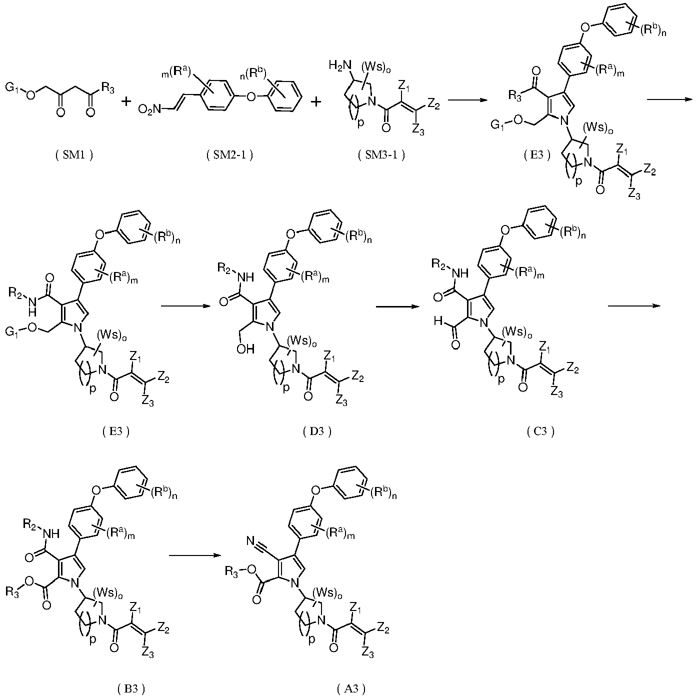 wherein R^{a}, R^{b}, R₁, R₂, R₃, Ws, Z₁, Z₂, Z₃, o, p, m and n are as defined in formula (Ib).

The present invention further provides a process for preparing a pharmaceutically acceptable salt of the compound of formula (Ia) by the reaction of the compound of formula (Ia) and an acid, wherein the acid is selected from the group consisting of an organic acid and inorganic acid, and preferably organic acid; the organic acid is selected from the group consisting of acetic acid, trifluoroacetic acid, oxalic acid, tartaric acid, maleic acid, fumaric acid, *p*-toluenesulfonic acid, benzenesulfonic acid, ethanesulfonic acid and methanesulfonic acid; and the inorganic acid is selected from the group consisting of hydrochloric acid, sulfuric acid and phosphoric acid.

### DETAILED DESCRIPTION OF THE INVENTION

In order to understand the present invention more easily, certain technical and scientific terms are specifically defined below. Unless otherwise definitely and obviously defined, all other technical and scientific terms used herein have the meaning commonly understood by those skilled in the art to which the present invention belongs.

The term "halogen" or "halogen atom" used in the present invention refers to fluorine, chlorine, bromine, iodine and the like.

The term "alkyl" used in the present invention refers to a linear or branched alkyl having 1 to 20 carbon atoms, including for example "C₁₋₆ alkyl", "C₁₋₄ alkyl" and the like. The specific examples of alkyl include, but are not limited to methyl, ethyl, *n*-propyl, isopropyl, *n*-butyl, isobutyl, *sec*-butyl, *tert*-butyl, *n*-pentyl, isopentyl, 2-methylbutyl, *neo*-pentyl, 1-ethylpropyl, *n*-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl and the like.

The term "alkylene" used in the present invention refers to a group formed by removing hydrogen atom(s) from an "alkyl", including for example "C₁₋₆ alkylene", "C₁₋₄ alkylene" and the like. The specific examples of alkylene include, but are not limited to methylene, ethylene, propylene, isobutylene, butylene, isobutylene, *sec*-butylene, *tert*-butylene, pentylene, isopentylene, *neo*-pentylene, *n*-hexylene, isohexylene and the like. The term "alkyl" is as defined above.

The term "alkenyl" used in the present invention refers to a linear or branched group having 2 to 20 carbon atoms and at least one carbon-carbon double bond, including for example "C₂₋₆ alkenyl", "C₂₋₄ alkenyl" and the like. The examples of alkenyl include, but are not limited to vinyl, propenyl, 2-butenyl, 2-pentenyl, 3-pentenyl, 2-hexenyl, 3-hexenyl and the like.

The term "alkynyl" used in the present invention refers to a linear or branched group having 2 to 20 carbon atoms and at least one carbon-carbon triple bond, including for example "C₂₋₆ alkynyl", "C₂₋₄ alkynyl" and the like. The examples of alkynyl include, but are not limited to ethynyl, propynyl, 2-butynyl, 2-pentynyl, 3-pentynyl, 4-methyl-2-pentynyl, 2-hexynyl, 3-hexynyl, 5-methyl-2-hexynyl and the like.

The term "haloalkyl" used in the present invention refers to a group derived from an "alkyl" in which one or more hydrogen atom(s) are substituted by one or more "halogen atom(s)", and the terms "halogen atom" and "alkyl" are as defined above.

The term "hydroxyalkyl" used in the present invention refers to a group derived from an "alkyl" in which one or more hydrogen atom(s) are substituted by one or more "hydroxy(s)", and the term "alkyl" is as defined above.

The term "alkoxy, haloalkoxy, alkylcarbonyl, formylalkyl, alkoxycarbonyl, formylalkoxy, alkylcarbonylamino, alkylaminocarbonyl, formylalkylamino, alkylamino, dialkylamino, alkylsulfonamido, alkylsulfonyl, alkenylcarbonyl or alkynylcarbonyl" used in the present invention refers to a group with a linkage form of alkyl-O-, haloalkyl-O-, alkyl-C(O)-, H-C(O)-alkyl-, alkyl-O-C(O)-, H-C(O)-alkyl-O-, alkyl-C(O)-NH-, alkyl-NH-C(O)-, H-C(O)-alkyl-NH-, alkyl-NH-, (alkyl)₂-N-, alkyl-S(O)₂-NH-, alkyl-S(O)₂-, alkenyl-C(O)- or alkynyl-C(O)-, wherein the terms "alkyl, haloalkyl, alkenyl, alkynyl" are as defined above.

The term "cycloalkyl" used in the present invention refers to a saturated or partially unsaturated monocyclic or polycyclic hydrocarbon group having 3 to 14 carbon atoms, preferably 3 to 12 carbon atoms or 5 to 12 carbon atoms, more preferably 3 to 8 carbon atoms, most preferably 5 to 6 carbon atoms, and the cycloalkyl is optimally cyclopropyl. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, and preferably cyclopropyl or cyclohexenyl. Polycyclic cycloalkyl includes a cycloalkyl having a spiro ring, fused ring or bridged ring.

The term "heterocyclyl" used in the present invention refers to a 3 to 14 membered saturated or partially unsaturated monocyclic or polycyclic hydrocarbon group, wherein at least one ring atoms are heteroatoms (for example nitrogen atoms, oxygen atoms or sulfur atoms), with the remaining ring atoms being carbon atoms. Optionally, the ring atoms (for example, carbon atoms, nitrogen atoms or sulfur atoms) of the cyclic structure can be oxidized. Preferably, the heterocyclyl has 3 to 12 ring atoms or 5 to 12 ring atoms wherein 1 to 4 heteroatoms, more preferably 3 to 8 ring atoms, and more preferably 5 to 6 ring atoms. Non-limiting examples of monocyclic heterocyclyl include pyrrolidinyl, piperidinyl, piperazinyl, morpholinyl, thiomorpholinyl, homopiperazinyl, pyranyl, tetrahydrofuranyl and the like. Polycyclic heterocyclyl includes a heterocyclyl having a spiro ring, fused ring or bridged ring.

The term "aryl" used in the present invention refers to a 6 to 14 membered all-carbon monocyclic ring or polycyclic fused ring (*i.e.* each ring in the system shares an adjacent pair of carbon atoms with another ring in the system) group having a conjugated *π*-electron system. The aryl is preferably a 6 to 8 membered aryl, more preferably phenyl, anthracenyl, phenanthryl, fluorenyl or indenyl, and most preferably phenyl.

The term "heteroaryl" used in the present invention refers to a 5 to 15 membered all-carbon monocyclic ring or fused polycyclic ring group having a conjugated *π*-electron system, and further having 1 to 4 heteroatoms selected from the group consisting of O, S and N. The heteroaryl is preferably a 5 to 8 membered heteroaryl, and more preferably a 5 or 6 membered heteroaryl. The specific examples of heteroaryl include, but are not limited to furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl and the like. The heteroaryl can also be fused to the ring of aryl, heterocyclyl or cycloalkyl.

The expression "carbon atoms, nitrogen atoms or sulfur atoms are oxidized" used in the present invention refers to the formation of a C=O, N=O, S=O or SO₂ structure.

The term "amide solvent" used in the present invention refers to a liquid compound in which the hydroxy group of the carboxy group of a carboxylic acid molecule is substituted with an amino or a hydrocarbon amino group (-NHR or -NR₂). It can also be regarded as a liquid compound in which the hydrogen on the nitrogen atom of an ammonia or amine molecule is substituted with an acyl. The specific examples of amide solvent include, but are not limited to *N,N*-dimethylformamide and *N,N*-dimethylacetamide.

The term "ester solvent" used in the present invention refers to a compound with less than 15 carbon atoms formed by a dehydration reaction between an organic acid and an alcohol or phenol, or a lower ester compound having a functional group -C(O)O- and less than 15 carbon atoms. The specific examples of ester solvent include, but are not limited to methyl acetate, ethyl acetate, dimethyl phthalate, butyl acetate or propyl acetate.

The term "ketone solvent" used in the present invention refers to a compound in which a carbonyl group (-C(O)-) is bonded to two hydrocarbon groups. Ketones can be classified into aliphatic ketones, alicyclic ketones, aromatic ketones, saturated ketones and unsaturated ketones, depending on the hydrocarbon groups in the molecule. The specific examples of ketone solvent include, but are not limited to acetone, methyl ethyl ketone, acetophenone, methyl isobutyl ketone or methyl pyrrolidone.

The term "ether solvent" used in the present invention refers to a chain compound or a cyclic compound having an ether bond -O- and 1 to 10 carbon atoms. The specific examples of ether solvent include, but are not limited to tetrahydrofuran, diethyl ether, propylene glycol methyl ether, ethylene glycol dimethyl ether, methyl *tert*-butyl ether or 1,4-dioxane.

The term "alcohol solvent" used in the present invention refers to a group derived from a "C₁₋₆ alkyl" in which one or more hydrogen atom(s) are substituted by one or more "hydroxy(s)", and the terms "hydroxy" and "C₁₋₆ alkyl" are as defined above. The specific examples of alcohol solvent include, but are not limited to methanol, ethanol, isopropanol, *n*-propanol, isopentanol or trifluoroethanol.

The term "nitrile solvent" used in the present invention refers to a group derived from a "C₁₋₆ alkyl" in which one or more hydrogen atom(s) are substituted by one or more "cyano(s)", and the terms "cyano" and "C₁₋₆ alkyl" are as defined above. The specific examples of nitrile solvent include, but are not limited to acetonitrile or propionitrile.

The term "halohydrocarbon solvent" used in the present invention refers to a group derived from a "C₁₋₆ alkyl" in which one or more hydrogen atom(s) are substituted by one or more "halogen atom(s)", and the terms "halogen atom" and "C₁₋₆ alkyl" are as defined above. The specific examples of halohydrocarbon solvent include, but are not limited to chloromethane, dichloromethane, chloroform or carbon tetrachloride.

The term "arene solvent" used in the present invention refers to a general term for a carbon ring compound and a derivative thereof, wherein the molecule has a conjugated system of a closed ring, and the number of π electrons conforms to the Huckel rule. The specific examples of arene solvent include, but are not limited to benzene, toluene, cumene or xylene.

The term "sulfoxide solvent" used in the present invention refers to a compound formed by the bond of a sulfinyl group (-SO-) to a hydrocarbon group. The specific examples of sulfoxide solvent include, but are not limited to dimethyl sulfoxide, diethyl sulfoxide or benzyl sulfoxide.

### Advantageous Effects of the Present Invention

Compared with the prior art, the technical solution for preparing the compound of formula (I) of the present invention has the following advantages:
(1) The starting materials and intermediates of the present invention are different from the prior art. The present invention provides a synthesis method with a completely different idea, wherein the starting materials and reactants are simple and easy to purchase.
(2) The yield is improved.
(3) The work-up is simple. The crude product of each step can be used directly in the next step without purification. This method is conducive to industrial production.

### Examples

The present invention will be further described with reference to the following examples, which should not be considered as limiting the scope of the present invention.

In the examples of the present invention, the experiment methods that do not specify the specific conditions are generally conducted in accordance with conventional conditions, or in accordance with conditions recommended by the material or product manufacturers. The reagents without a specific source are commercially available conventional reagents.

The structures of the compounds are identified by nuclear magnetic resonance (NMR) and/or mass spectrometry (MS). NMR shifts (δ) are given in 10⁻⁶ (ppm). NMR are determined by a Bruker AVANCE-400 machine. The solvent for determination is heavy water with sodium hydroxide (CDCl₃), and the internal standard is tetramethylsilane (TMS).

High performance liquid chromatography (HPLC) is determined on a Waters Alliance 2695 high performance liquid chromatograph spectrometer and an Agilent 1200 series liquid chromatograph spectrometer, with octadecylsilane bonded silica gel as the column packing.

### Example 1. Preparation of (R)-4-amino-1-(1-(but-2-ynoyl)pyrrolidin-3-yl)-3-(4-(2,6-difluorophenoxy)phenyl)-1,6-dihydro-7H-pyrrolo[2,3-d]pyridazin-7-one

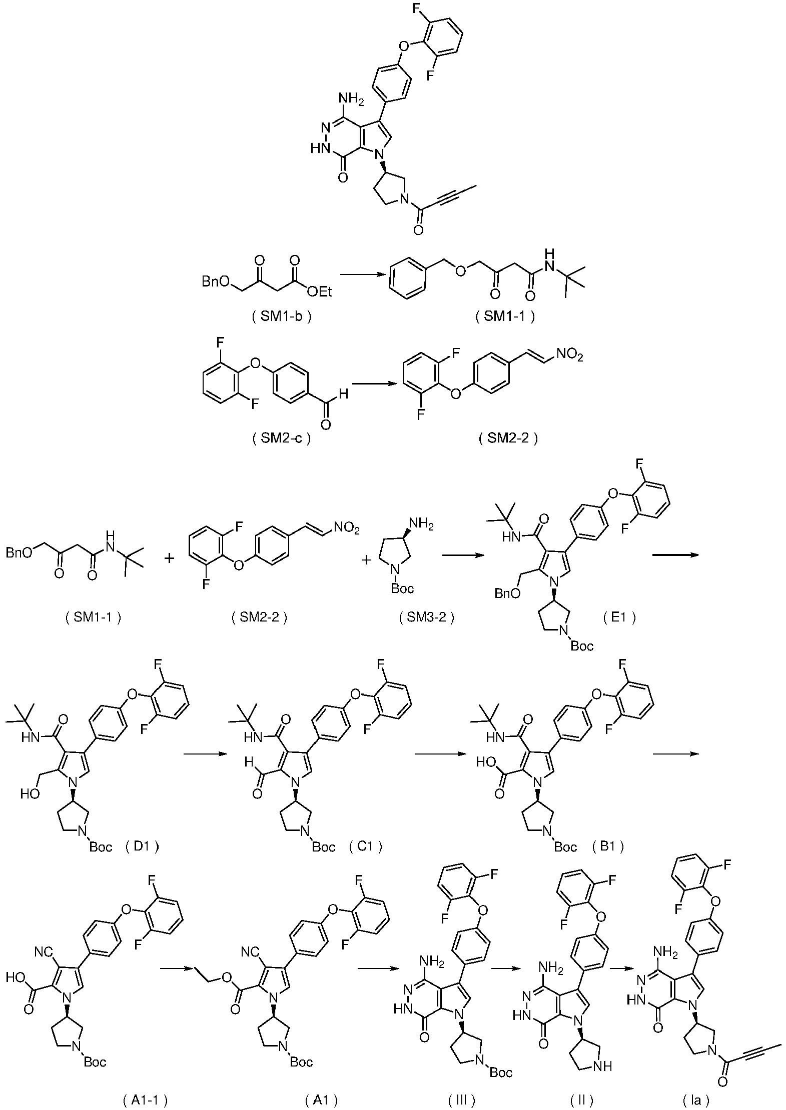

### Step 1. Synthesis of the compound of formula (SM1-1)

Compound **SM1-b** (20 g), *tert*-butylamine (9.91 g) and 4-dimethylaminopyridine (5.17 g) were added to toluene (400 mL) under a nitrogen atmosphere. The reaction solution was heated to reflux and stirred for 6 hours. The reaction was stopped after completion, and the reaction solution was concentrated. The resulting residues were purified by column chromatography (eluent: petroleum ether:ethyl acetate = 5:1) to obtain the target compound (16.6 g, yield: 74.5%).

### Step 2. Synthesis of the compound of formula (SM2-2)

Acetic acid (2600 mL) and ammonium acetate (411.42 g) were added to a reaction flask, and stirred to dissolve completely. A solution of compound **SM2-c** (500 g) in nitromethane (912.2 g) was added, and the reaction solution was heated to 90°C with an oil bath and kept for 5 hours. The reaction was stopped, and the oil bath was removed. The reaction solution was added with water (5.2 L) to precipitate a solid. The system was stirred for crystallization for 2 hours, and then filtrated to give 662 g of crude product. The crude product was added to isopropanol (2.5 L), and heated to reflux to dissolve completely. The resulting solution was cooled to room temperature, stirred overnight, filtrated and dried to obtain the a product (443 g, yield: 74.9%).

### Step 3. Synthesis of the compound of formula (E1)

Compound **SM1-1** (2.28 g), compound **SM3-2** (1.88 g), compound **SM2-2** (2 g), nitromethane (4.4 g) and ferric chloride (234 mg) were added to toluene (50 mL) under a nitrogen atmosphere. The reaction solution was heated to reflux and stirred for 2 hours. After completion of the reaction, the reaction solution was cooled to room temperature, and purified by column chromatography (eluent: petroleum ether:ethyl acetate = 2:1) to obtain a product (3.4 g, yield: 69.9%).

### Step 4. Synthesis of the compound of formula (D1)

Compound **E1** (30 g) and methanol (300 mL) were added to a reaction flask and stirred, then palladium hydroxide (9.0 g) was added. The reaction system was purged with hydrogen three times, and the reaction solution was heated to 50°C under hydrogen atmosphere and stirred. After completion of the reaction, the reaction solution was cooled to room temperature, and filtrated through celite. The filtrate was concentrated to obtain 27.0 g of crude product, which was used directly in the next oxidation reaction.

### Step 5. Synthesis of the compound of formula (C1)

Compound **D1** (27.0 g) and dimethyl sulfoxide (270 mL) were added to a reaction flask and stirred to dissolve, followed by an one-time addition of S-IBX (56.5 g, IBX content ∼47%). The reaction solution was stirred at 40°C for 1 hour. After completion of the reaction, the reaction solution was cooled naturally, followed by the addition of methyl *tert*-butyl ether (200 mL). The reaction solution was stirred well, and then filtrated through celite to remove the solid. The filtrate was extracted with methyl *tert*-butyl ether (200 mL×3). The organic phases were combined, washed with water (150 mL×3) three times, dried over anhydrous sodium sulfate, filtrated and concentrated to obtain a product (26.0 g, yield: 96.6%).

### Step 6. Synthesis of the compound of formula (B1)

Compound **C1** (26.0 g), *tert*-butanol (500 mL) and isopentene (57.6 mL) were added to a reaction flask and stirred to dissolve. Sodium chlorite (20.7 g) and sodium dihydrogen phosphate (12.4 g) were dissolved in water (100 mL), and the resulting solution was added dropwise to the above reaction solution. After completion of the addition, the reaction solution was stirred at 20°C. After completion of the reaction, the reaction solution was concentrated to remove most of the *tert*-butanol. The residues were added with water (300 mL), and then extracted with methyl *tert*-butyl ether (300 mL×3). The organic phases were combined, washed with water (200 mL), dried over anhydrous sodium sulfate, filtrated and concentrated to obtain a product (28.3 g, yield: 105.8%).

### Step 7. Synthesis of the compound of formula (A1-1)

Compound **B1** (28.3 g) and dichloromethane (400 mL) were added to a reaction flask. Trifluoroacetic anhydride (20.4 g) was dissolved in dichloromethane (160 mL), and the resulting solution was added dropwise to the above reaction system. After completion of the addition, the reaction solution was reacted at room temperature. After completion of the reaction, 200 mL of water was added, and the solution was stirred well and separated into layers. The aqueous phase was extracted with dichloromethane (200 mL×2). The organic phases were combined, washed with water until neutral, dried over anhydrous magnesium sulfate, filtrated and concentrated to obtain a product (25.0 g, yield: 100%).

25.0 g of the resulting crude product was dissolved in methyl *tert*-butyl ether (200 mL). *Tert*-butylamine (6.2 mL) was diluted in methyl *tert*-butyl ether (50 mL), and the resulting solution was slowly added dropwise to the above system. The solution was stirred at room temperature for 5 hours to slowly precipitate solid, and then filtrated. The filter cake was washed with methyl tert-butyl ether, and dried under vacuum to obtain a product (21.5 g, yield: 75%, purity: 94.1%).

### Step 8. Synthesis of the compound of formula (A1)

The *tert*-butylamine salt of compound **A1-1** (21.5 g), potassium carbonate (15.3 g) and *N,N*-dimethylformamide (200 mL) were added to a reaction flask. Iodoethane (8.8 mL) was added under an ice bath. After completion of the addition, the ice bath was removed, and the reaction solution was reacted at 30°C until the raw materials were consumed completely. The reaction mixture was added with water (200 mL), and then extracted with methyl *tert*-butyl ether (200 mL×3). The organic phases were combined, washed with water (100 mL×3), dried over anhydrous sodium sulfate, filtrated and concentrated to obtain a product (18.5 g, yield: 91%).

### Step 9. Synthesis of the compound of formula (III)

Compound **A1** (6.0 g), hydrazine hydrate (35 mL) and ethanol (60 mL) were added to a reaction flask. The system was purged with nitrogen, and the reaction solution was heated to reflux. After completion of the reaction, the reaction solution was naturally cooled to room temperature, and concentrated under reduced pressure to remove most of the ethanol solvent. The residues were added with saturated sodium chloride aqueous solution (100 mL), and then extracted with ethyl acetate (100 mL×3). The organic phases were combined, washed with water (100 mL), dried over anhydrous sodium sulfate, filtrated and concentrated to remove the solvent. The residues were purified by silica gel column chromatography (eluent: petroleum ether:ethyl acetate = 2:1) to obtain a product (4.5 g, yield: 78.7%).

### Step 10. Synthesis of the compound of formula (II)

Compound **III** (4.5 g) and methanol (50 mL) were added to a reaction flask and stirred to dissolve. 6 N hydrochloric acid (40 mL) was added under an ice bath. The ice bath was removed, and the reaction solution was reacted at 25°C until the raw materials were consumed completely. The reaction solution was concentrated under reduced pressure to remove most of the solvent, and the pH was adjusted with NaOH to about 10. The solution was extracted with dichloromethane (150 mL×3). The organic phases were combined, washed with water (100 mL×2), dried over anhydrous magnesium sulfate, filtered and concentrated to remove the solvent and obtain a product (3.2 g, purity >99%, yield: 88.0%), which was used directly in the next condensation reaction.

### Step 11. Synthesis of the compound of formula (Ia)

Compound **II** (3.2 g) and dichloromethane (33 mL) were added to a reaction flask, and then butynoic acid (0.95 g), EDCI (2.9 g) and triethylamine (3.2 mL) were added under an ice bath. The reaction solution was stirred at 20°C until the raw materials were consumed completely. The reaction was quenched by adding water (100 mL), and the aqueous phase was extracted with dichloromethane (100 mL×3). The organic phases were combined, dried over anhydrous sodium sulfate, filtrated and concentrated to remove the solvent. The residues were purified by silica gel column chromatography (eluent: petroleum ether:ethyl acetate = 2:1) to obtain a product (3.2 g, yield: 86.0%, purity: 98.3%).

2.5 g of the above product was dissolved in acetonitrile (25 mL) at room temperature, and heated to 70°C to dissolve. The solution was naturally cooled to room temperature to precipitate a large amount of solid. The mixture was stirred for 2 hours and then filtrated, and the filter cake was washed with acetonitrile to give the target product (1.9 g, yield: 76%).
¹H-NMR (400MHz, CDCl₃) *δ* 11.5(br, 1H),7.38-7.40(d, 2H), 7.16-7.24(m, 1H), 7.02-7.08(m, 5H), 6.34-6.38(m, 1H), 5.30-5.32(br, 2H), 4.19-4.24(m, 0.5H), 3.69-3.98(m, 3.5H), 2.53-2.58(m, 1H), 2.31-2.37(m, 1H), 1.96-2.02(d, 3H).

### Example 2. Preparation of (R)-1-(1-acryloylpiperidin-3-yl)-4-amino-3-(4-(2,6-difluorophenoxy)phenyl)-1,6-dihydr o-7H-pyrrolo[2,3-d]pyridazin-7-one

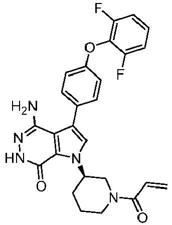

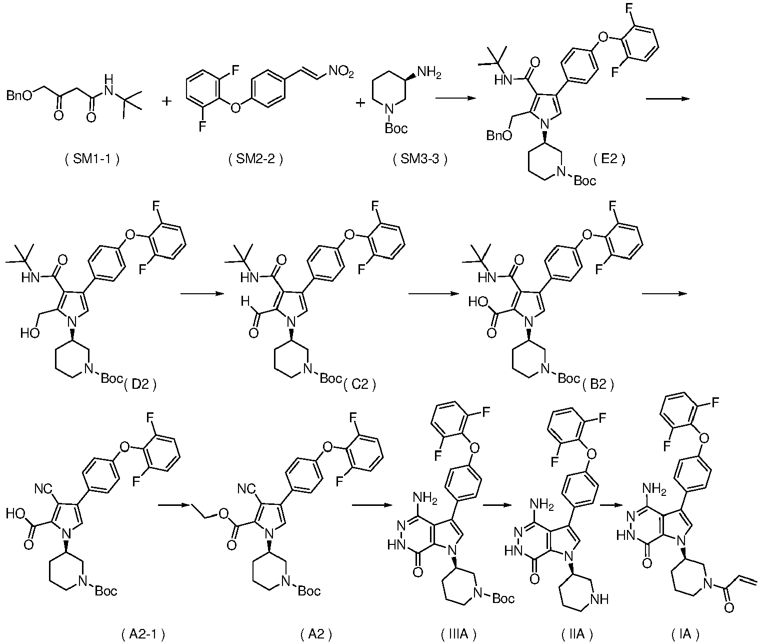

The target compound IA was obtained in accordance with the same preparation method of Example 1 except for replacing the reactant **SM3-2** with compound **SM3-3.**

## Claims

1. A compound of formula (E), a salt thereof, or a stereoisomer thereof, 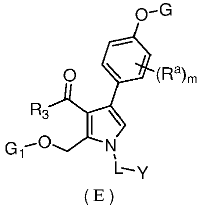 wherein,
R^{a} is selected from the group consisting of hydrogen, halogen, hydroxy, nitro, cyano, carboxy, amino, alkyl, haloalkyl, haloalkoxy and alkoxy;
R₃ is selected from the group consisting of hydrogen, halogen, alkyl, -OR₁, -NHR₂, -NR₂R₂ and alkylsulfonamido;
R₁ is selected from the group consisting of hydrogen, alkyl, alkylcarbonyl, cycloalkyl, cycloalkylcarbonyl, heterocyclyl, heterocyclylcarbonyl, aryl, arylcarbonyl, heteroaryl and heteroarylcarbonyl;
R₂ is selected from the group consisting of hydrogen, alkyl, alkylamino, alkylcarbonyl, alkoxycarbonyl, alkylaminocarbonyl, cycloalkyl, cycloalkylcarbonyl, heterocyclyl, heterocyclylcarbonyl, aryl, arylcarbonyl, heteroaryl and heteroarylcarbonyl;
G is selected from the group consisting of aryl, heteroaryl, cycloalkyl and heterocyclyl, which are optionally substituted by a substituent, wherein the substituent is selected from the group consisting of hydrogen, halogen, hydroxy, nitro, cyano, carboxy, amino, alkyl, alkoxy, alkylamino, hydroxyalkyl, dialkylamino, alkylcarbonyl, formylalkyl, alkoxycarbonyl, formylalkoxy, alkylcarbonylamino, alkylaminocarbonyl, alkylsulfonyl, alkenyl, alkenylcarbonyl, alkynyl and alkynylcarbonyl;
G₁ is selected from the group consisting of hydrogen and a hydroxy protecting group;
L is an alkylene or absent;
Y is selected from the group consisting of cycloalkyl, heterocyclyl, aryl and heteroaryl, which are optionally substituted by a substituent, wherein the substituent is selected from the group consisting of halogen, cyano, alkylcarbonyl, alkoxycarbonyl, alkylcarbonylamino, alkylsulfonyl, alkylsulfonamido, alkyl, cycloalkyl, alkenyl, alkenylcarbonyl, alkynyl and alkynylcarbonyl, Y is preferably a 3 to 8 membered heterocyclyl, and more preferably pyrrolidinyl or piperidinyl;
m is 0, 1, 2 or 3.

2. The compound according to claim 1, which is a compound of formula (E1) or (E2), 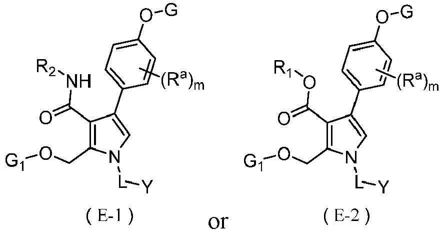 wherein R^{a}, R₁, R₂, G, G₁, L, Y and m are as defined in claim 1.

3. The compound according to claims 1 or 2,which is selecting from the group consisting of

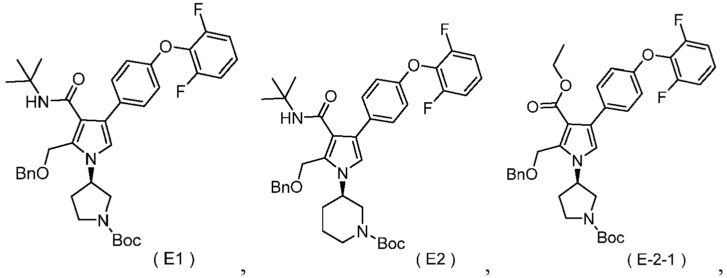

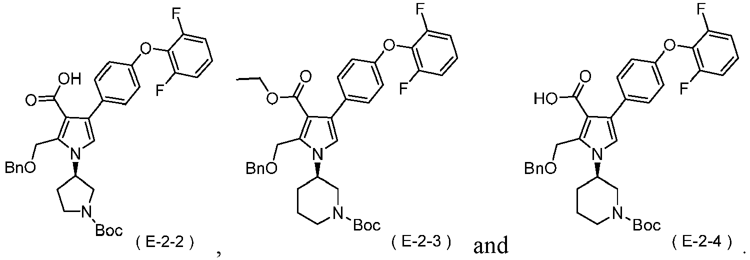

4. A method for preparing the compound of formula (E) or a stereoisomer thereof, **characterized in that** the method comprises a step of reacting a compound of formula (SM1) or a stereoisomer thereof, a compound of formula (SM2) or a stereoisomer thereof, and a compound of formula (SM3) or a stereoisomer thereof to obtain the compound of formula (E) or a stereoisomer thereof, 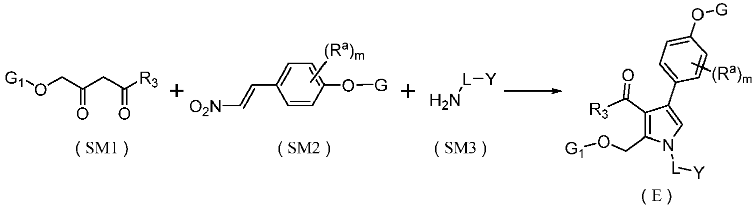 wherein R^{a}, R₃, G, G₁, L, Y and m are as defined in claim 1.

5. The method according to claim 4, **characterized in that** R₃ is not -OR₁, and the method further comprises a step of

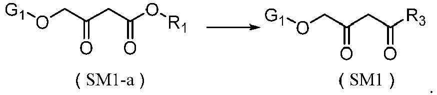

6. The method according to claim 5, **characterized in** further comprises a step of

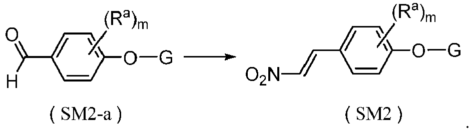

7. A method for preparing the compound of formula (E1) or a stereoisomer thereof, **characterized in that** the method comprises a step of

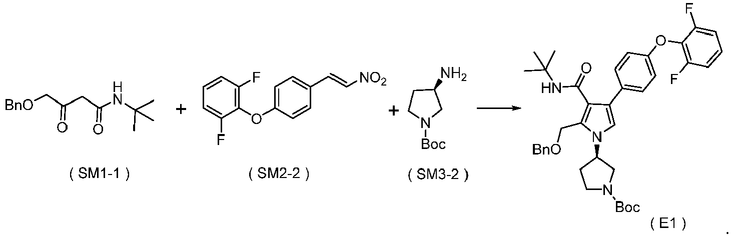

8. The method according to claim 7, **characterized in** further comprises a step of

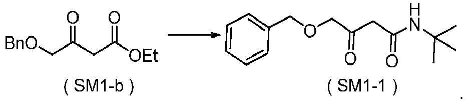

9. The method according to claim 8, **characterized in** further comprises a step of

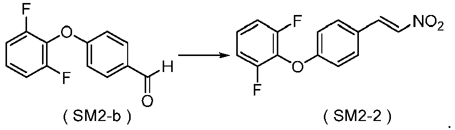

10. A compound of formula (SM1), a salt thereof, or a stereoisomer thereof, 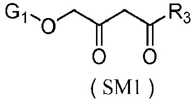 wherein R₃ and G₁ are as defined in claim 1.

11. The compound according to claim 10, which is selected from the group consisting of

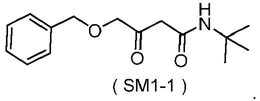

12. A compound of formula (SM2), a salt thereof, or a stereoisomer thereof, 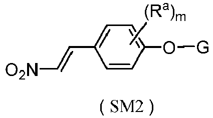 wherein R^{a}, G and m are as defined in claim 1.

13. The compound according to claim 12, which is

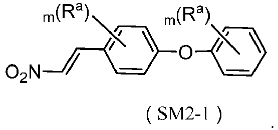

14. The compound according to claim 13, which is selected from the group consisting of

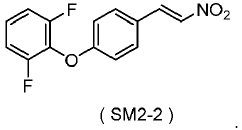

15. A method for preparing the compound of formula (SM1) or a stereoisomer thereof, **characterized in that** the method comprises a step of reacting a compound of formula (SM1-a) to obtain the compound of formula (SM1) or a stereoisomer thereof, 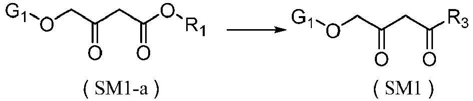 wherein R₁, R₃ and G₁ are as defined in claim 1, and R₃ is not -OR₁.

16. The method according to claim 15, which is

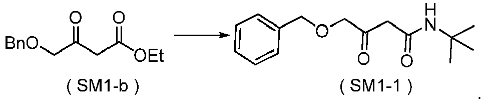

17. A method for preparing the compound of formula (SM2) or a stereoisomer thereof, **characterized in that** the method comprises a step of reacting a compound of formula (SM2-a) or a stereoisomer thereof to obtain the compound of formula (SM2) or a stereoisomer thereof, 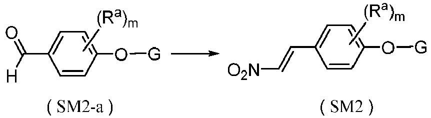 wherein R^{a}, G and m are as defined in claim 1.

18. The method according to claim 17, which is

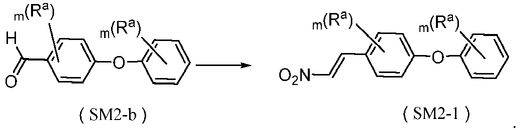

19. The method according to claim 18, which is

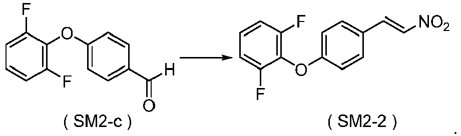

20. A compound of formula (B), a salt thereof, or a stereoisomer thereof, 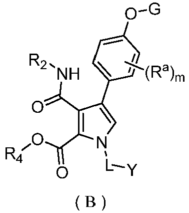
wherein R^{a}, R₂, G, L, Y and m are as defined in claim 1;
R₄ is selected from the group consisting of hydrogen, alkyl, alkylcarbonyl, alkoxycarbonyl, alkylaminocarbonyl, alkylsulfonyl, cycloalkyl, heterocyclyl, aryl and heteroaryl.

21. The compound according to claim 20, which is selected from the group consisting of

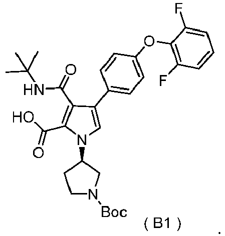

22. A method for preparing the compound of formula (B) or a stereoisomer thereof, **characterized in that** the method comprises a step of: 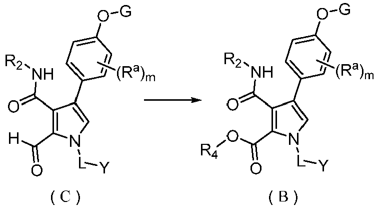 wherein R^{a}, R₂, G, L, Y and m are as defined in claim 1; and R₄ is as defined in claim 20.

23. The method according to claim 22, **characterized in** further comprises a step of

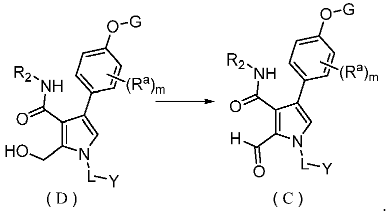

24. The method according to claim 23, **characterized in** further comprises steps of 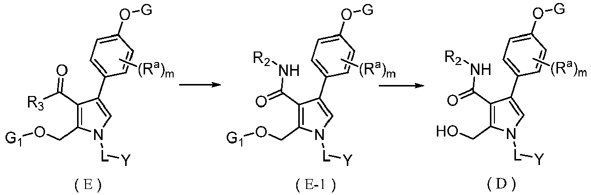 wherein G₁ and R₃ are as defined in claim 1, R₃ is not -NHR₂; and the method for preparing the compound of formula (E) is as defined in claims 4, 5 or 6.

25. A method for preparing the compound of formula (B1) or a stereoisomer thereof, **characterized in that** the method comprises a step of

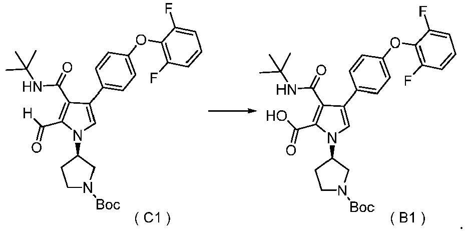

26. The method according to claim 25, **characterized in** further comprises a step of

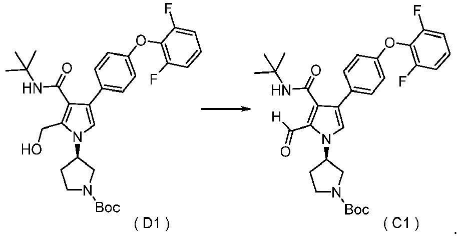

27. The method according to claim 26, **characterized in** further comprises a step of 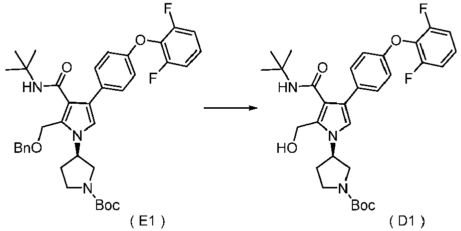 wherein the method for preparing the compound of formula (E1) is as defined in claims 7, 8 or 9.

28. A method for preparing the compound of formula (A) or a stereoisomer thereof, **characterized in that** the method comprises a step of: 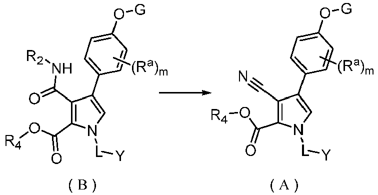 wherein R^{a}, R₂, G, L, Y and m are as defined in claim 1; R₄ is as defined in claim 20; and the method for preparing the compound of formula (B) is as defined in claims 22, 23 or 24.

29. A method for preparing the compound of formula (A1) or a stereoisomer thereof, **characterized in that** the method comprises the steps of 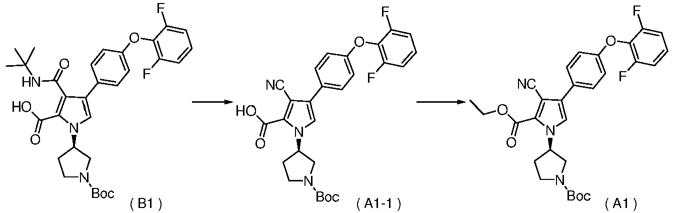 wherein the method for preparing the compound of formula (B1) is as defined in claims 25, 26 or 27.

30. A method for preparing the compound of formula (I) or a stereoisomer thereof, **characterized in that** the method comprises the steps of reacting a compound of formula (B) or a stereoisomer thereof to obtain a compound of formula (A) or a stereoisomer thereof, and reacting the compound of formula (A) or a stereoisomer thereof to obtain the compound of formula (I) or a stereoisomer thereof 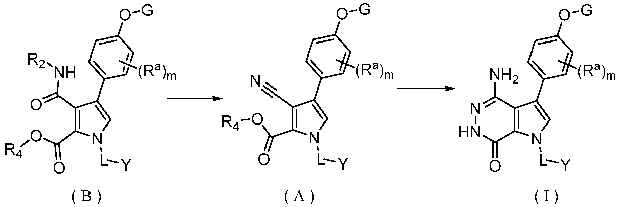 wherein R^{a}, R₂, G, L, Y and m are as defined in claim 1; R₄ is as defined in claim 20; and the method for preparing the compound of formula (B) is as defined in claims 22, 23 or 24.

31. A method for preparing the compound of formula (Ia) or a stereoisomer thereof, **characterized in that** the method comprises the steps of reacting a compound of formula (B1) to obtain a compound of formula (A1-1), reacting the compound of formula (A1-1) to obtain a compound of formula (A1), reacting the compound of formula (A1) to obtain a compound of formula (III), reacting the compound of formula (III) to obtain a compound of formula (II), and reacting the compound of formula (II) to obtain the compound of formula (Ia), 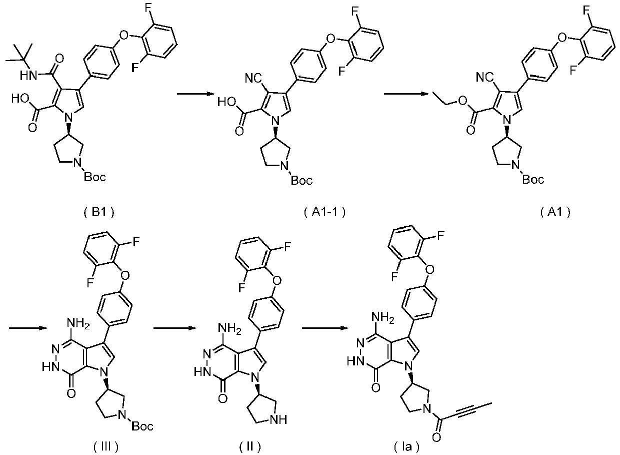 wherein the method for preparing the compound of formula (B1) comprises the method according to claims 25, 26 or 27.

32. A method for preparing the compound of formula (Ia) or a stereoisomer thereof, **characterized in that** the method comprises the steps of

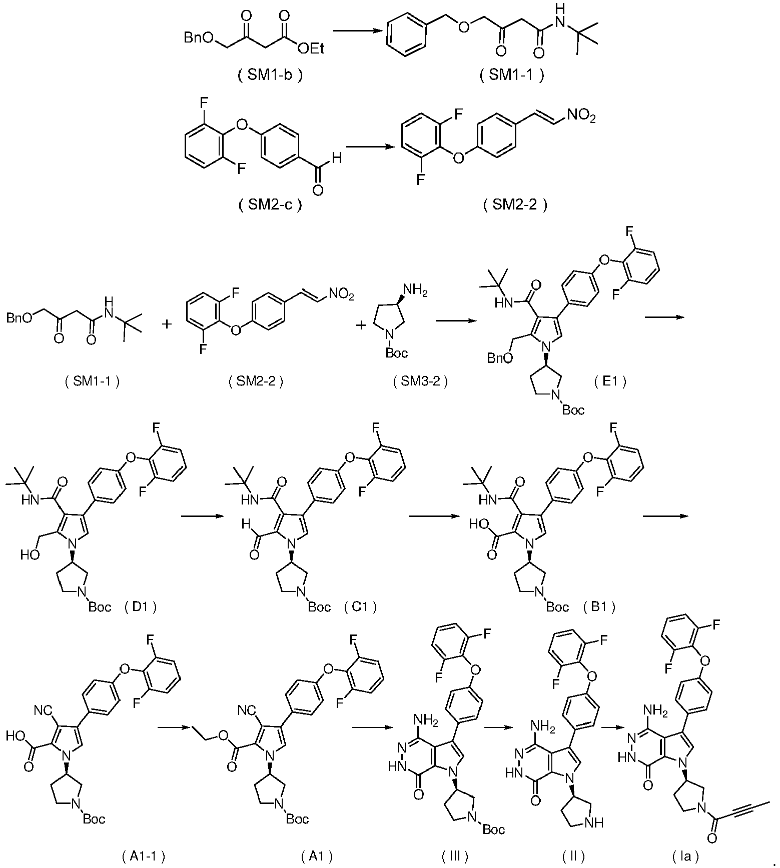

33. A method for preparing the compound of formula (A1) or a stereoisomer thereof, **characterized in that** the method comprises the steps of

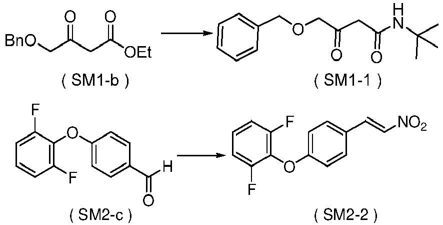

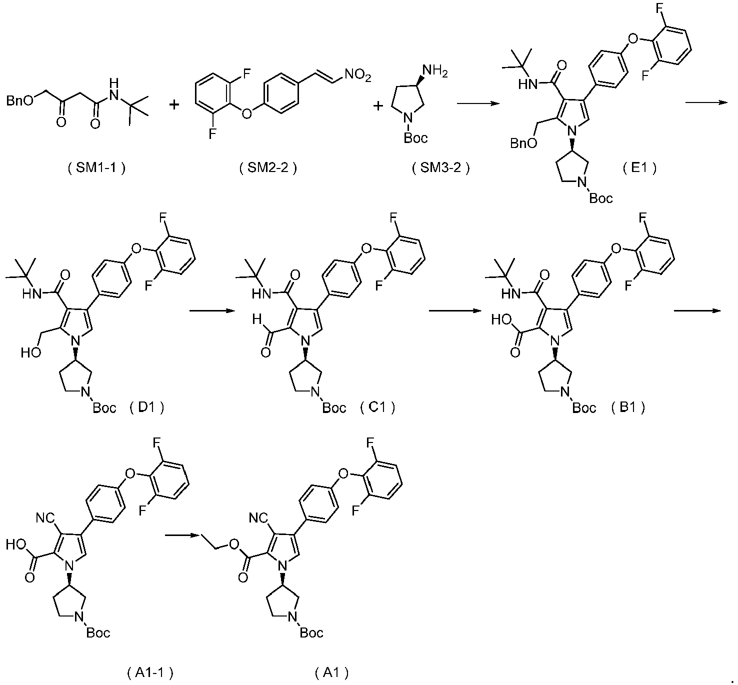

34. A compound of formula (C), a salt thereof, or a stereoisomer thereof, 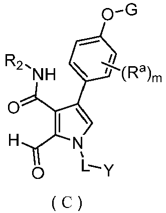 wherein R^{a}, R₂, G, L, Y and m are as defined in formula (E).

35. The compound according to claim 34, which is selected from the group consisting of
